(19) Europäisches Patentamt
European Patent Office
Office européen des brevets

(11) Veröffentlichungsnummer: **0 354 571**
**B1**

(12) **EUROPÄISCHE PATENTSCHRIFT**

(45) Veröffentlichungstag der Patentschrift: **09.01.91**

(21) Anmeldenummer: **89114821.5**

(22) Anmeldetag: **10.08.89**

(51) Int. Cl.$^5$: **C 07 C 251/48,** A 01 N 37/50, C 07 D 215/50, C 07 D 215/54, C 07 D 215/48, C 07 D 333/38, C 07 D 333/24, C 07 D 307/68, C 07 D 207/327, C 07 D 207/34, C 07 D 231/12

# (54) Oximether, Verfahren zu ihrer Herstellung und diese Verbindungen enthaltende Fungizide.

(30) Priorität: **12.08.88 DE 3827361**

(43) Veröffentlichungstag der Anmeldung:
**14.02.90 Patentblatt 90/07**

(45) Bekanntmachung des Hinweises auf die Patenterteilung:
**09.01.91 Patentblatt 91/02**

(84) Benannte Vertragsstaaten:
**AT BE CH DE ES FR GB GR IT LI NL SE**

(56) Entgegenhaltungen:
**EP-A-0 253 213**
**EP-A-0 254 426**

(73) Patentinhaber: **BASF Aktiengesellschaft**
**Carl-Bosch-Strasse 38**
**D-6700 Ludwigshafen (DE)**

(72) Erfinder: **Wenderoth, Bernd, Dr.**
**Schwalbenstrasse 26**
**D-6840 Lampertheim (DE)**
Erfinder: **Schuetz, Franz, Dr.**
**Budapester Strasse 45**
**D-6700 Ludwigshafen (DE)**
Erfinder: **Brand, Siegbert, Dr.**
**Hegelstrasse 39**
**D-6940 Weinheim (DE)**
Erfinder: **Sauter, Hubert, Dr.**
**Neckarpromenade 20**
**D-6800 Mannheim 1 (DE)**
Erfinder: **Ammermann, Eberhard, Dr.**
**Sachsenstrasse 3**
**D-6700 Ludwigshafen (DE)**
Erfinder: **Lorenz, Gisela, Dr.**
**Erlenweg 13**
**D-6730 Neustadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 354 571 B1

**Beschreibung**

Die vorliegende Erfindung betrifft neue Oximetherderivate, ihre Herstellung, diese enthaltende Fungizide und ihre Verwendung als Fungizide. Es ist bekannt, Oximether zum Beispiel das 2-Benzyloxyphenyl-glyoxylsäuremethylester-O-methyloxim als Fungizide zu verwenden (EP 253 213, 254 426). Ihre fungizide Wirkung ist jedoch oft nicht ausreichend.

Es wurde nun gefunden, daß neue fungizide Oximether der Formel I

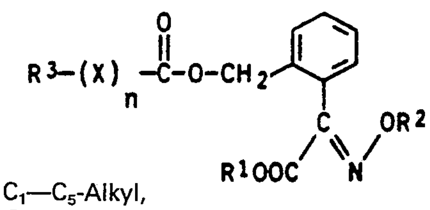

(I)

in der

$R^1$ und $R^2$ Wasserstoff oder $C_1$—$C_5$-Alkyl,

$R^3$ Wasserstoff, Halogen, Cyano, Aryl, Aryloxy, wobei der aromatische Ring gegebenenfalls substituiert ist durch $C_1$—$C_6$-Alkyl, $C_2$—$C_4$-Alkenyl, $C_1$—$C_2$-Halogenalkyl, $C_1$—$C_6$-Alkoxy, $C_1$—$C_4$-Alkoxy-$C_1$—$C_4$-alkyl, Aryl, Aryl-$C_1$—$C_2$-alkyl, Aryloxy, Ayloxy-$C_1$—$C_4$-alkyl, Aryloxy-$C_1$—$C_4$-alkoxy, Halogenaryloxy-$C_1$—$C_4$-alkoxy, Halogen, Halogen-$C_1$—$C_4$-alkoxy, $C_1$—$C_4$-Alkylthio, Thiocyanato, Cyano, Nitro, oder $R^3$ Heteroaryl, Adamantyl, Fluorenyl oder einen $C_3$—$C_7$-Cycloalkylrest oder $C_5$—$C_6$-Cycloalkenylrest bedeutet, wobei diese Reste gegebenenfalls substituiert sind durch $C_1$—$C_4$-Alkyl (Methyl, Ethyl), Halogen, (Chlor, Brom), $C_1$—$C_2$-Halogenalkyl, (Trifluormethyl, Tetrabromethyl, Dichlor-di-bromethyl), $C_3$—$C_4$-Alkenyl (Methylvinyl, Dimethylvinyl), $C_2$—$C_4$-Halogenalkenyl (Dichlorvinyl, Dichlorbutadienyl, Difluorvinyl, Trifluormethylvinyl), Acetyl, Methoxycarbonyl-$C_3$—$C_4$-Alkenyl, (Methyl-Methoxycarbonylvinyl), Cyclopentylidenmethyl, Halogenphenyl (Chlorphenyl), Phenyl, $C_1$—$C_2$-Alkoxyphenyl (Ethoxyphenyl), $C_1$—$C_4$-Alkylphenyl (tert. Butylphenyl),

X einen geradkettigen oder verzweigten, gegebenenfalls durch Halogen oder Hydroxy substituierten, gegebenenfalls ungesättigten $C_1$—$C_{12}$-Alkylenrest bedeutet und

n die Zahlen 0 oder 1 bedeutet,

eine ausgezeichnete fungizide Wirkung haben.

Die in der Formel I aufgeführten Reste können beispielsweise folgende Bedeutung haben:

$R^1$ und $R^2$ sind gleich oder verschieden und stehen z.B. für Wasserstoff, $C_1$—$C_5$-Alkyl, Methyl, Ethyl, Propyl, i-Propyl, Butyl, Pentyl.

$R^3$ kann z.B. Wasserstoff, Halogen (z.B. Fluor, Chlor, Brom), Cyano, Aryl (Phenyl, Naphthyl) oder Aryloxy (Phenoxy) sein, wobei der aromatische Ring gegebenenfalls durch bis zu drei der folgenden Reste substituiert ist: $C_1$—$C_6$-Alkyl (z.B. Methyl, Ethyl, n- oder iso-Propyl, n-, iso-, sec.-oder tert.-Butyl, n-, iso-, sec.-, tert.- oder neo-Pentyl, Hexyl), $C_2$—$C_4$-Alkenyl (z.B. Vinyl, Allyl), $C_1$—$C_2$-Halogenalkyl (z.B. Difluormethyl, Trifluormethyl), $C_1$—$C_6$-Alkoxy (z.B. Methoxy, Ethoxy, iso-Propoxy, tert.-Butoxy), $C_1$—$C_4$-Alkoxy-$C_1$—$C_4$-alkyl (z.B. Methoxymethyl), Aryl (z.B. Phenyl), Aryl-$C_1$—$C_2$-alkyl (z.B. Benzyl), Aryloxy (z.B. Phenoxy), Aryloxy-$C_1$—$C_4$-alkyl (z.B. Phenoxymethyl, Phenoxyethyl), Aryloxy-$C_1$—$C_4$-alkoxy, Halogenaryloxy-$C_1$—$C_4$-alkoxy, (z.B. Phenoxymethoxy, Phenoxyethoxy, Phenoxypropoxy, 2-Chlor-phenoxy-ethoxy, 4-Chlor-phenoxy-ethoxy), Halogen (z.B. Fluor, Chlor, Brom, Jod), Halogen-$C_1$—$C_4$-alkoxy (z.B. 1,1,2,2-Tetrafluoroethoxy), $C_1$—$C_4$-Alkylthio (z.B. Methylthio), Thiocyanato, Cyano, Nitro.

$R^3$ kann ferner bedeuten:

Heteroaryl (z.B. Pyridyl, Chinolyl, Pyrimidinyl, Furyl, Pyrrolyl), wobei das heterocyclische System gegebenenfalls bis zu dreifach durch Aryl (Phenyl), $C_1$—$C_4$-Alkyl (z.B. Methyl, Propyl, Butyl), Acetyl und/ oder Halogen (z.B. Fluor, Chlor) substituiert sein kann, $R^3$ bedeutet ferner $C_3$—$C_7$-Cycloalkyl, $C_5$—$C_6$-Cycloalkenyl, (z.B. Cyclopropyl, Cyclobutyl, Cyclopentyl, Cyclopentenyl, Cyclohexyl, 1-Methylcyclohexyl, Cyclohexenyl, Cycloheptyl), 1-Adamantyl, 9-Fluorenyl oder einen Cyclopropylrest, der bis zu vierfach substituiert ist durch $C_1$—$C_4$-Alkyl (Methyl, Ethyl), Halogen (Chlor, Brom), $C_1$—$C_2$-Halogenalkyl (Trifluormethyl, Tetrabromethyl, Dichlor-dibromethyl), $C_3$—$C_4$-Alkenyl (Methylvinyl, Dimethylvinyl), $C_2$—$C_4$-Halogenalkenyl (Dichlorvinyl, Diochlorbutadienyl, Difluorvinyl, Trifluormethylvinyl), Methoxycarbonyl-$C_3$—$C_4$-Alkenyl (Methyl-Methoxycarbonylvinyl), Cyclopentylidenmethyl, Phenyl, Halogenphenyl (Chlorphenyl, Bromphenyl), $C_1$—$C_2$-Alkoxyphenyl (Methoxyphenyl, Ethoxyphenyl), $C_1$—$C_4$-Alkylphenyl (tert. Butylphenbyl), wie zum Beispiel:

2,2-Dimethyl-3-(2',2'-dimethylvinyl)-cyclopropyl (A1)
2,2-Dimethyl-3-(2',2'-dichlorvinyl)-cyclopropyl (A2)
2,2-Dimethyl-3-(2',2'-dibromvinyl)-cyclopropyl (A3)
2,2-Dimethyl-3-(2'-trifluormethyl-2'-chlorvinyl)-cyclopropyl (A4)
2,2-Dichlor-3,3-dimethyl-cyclopropyl (A5)
2,2,3,3-Tetramethyl-cyclopropyl (A6)
2,2-Dimethyl-3-(2',2'-difluorvinyl)-cyclopropyl (A7)
2,2-Dimethyl-3-(2'-trifluormethyl-2'fluorvinyl)-cyclopropyl (A8)
2,2-Dimethyl-3-(2'-methyl-2'-methoxycarbonylvinyl)-cyclopropyl (A9)
2,2-Dimethyl-3-(4',4'-dichlorbutadienyl)-cyclopropyl (A10)

2,2-Dimethyl-3-(1'-brom-2',2',2'-tribromethyl)-cyclopropyl (A11)
2,2-Dimethyl-3-(1'-brom-2',2'-dichlor-2'-bromethyl)-cyclopropyl (A12)
1-(2',4'-Dichlorphenyl)-cyclopropyl (A13)
1-(4'-Chlorphenyl)-cyclopropyl (A14)
1-(4'-Ethoxyphenyl)-2,2-dichlor-cyclopropyl (A15)
2,2-Dimethyl-3-(4'-tert.-Butylphenyl)cyclopropyl (A16)
1-Methyl-2,2-dichlorcyclopropyl (A17)

X kann beispielsweise bedeuten:

einen geradkettigen $C_1$—$C_{12}$-Alkylenrest (z.B. Methylen, Ethylen, Propylen, Butylen, Pentylen, Hexylen, Heptylen), einen verzweigten $C_1$—$C_{12}$-Alkylenrest (z.B. Methylmethylen, Dimethylmethylen, Ethylmethylen, n- oder iso-Propylmethylen, Methylethylen, Methylpropylen, Dimethylpropylen, Ethylpropylen, Methylbutylen, Dimethylbutylen, Ethylbutylen, n- oder iso-Propylbutylen, Methylpentylen, Dimethylpentylen, Trimethylpentylen, Methylhexylen, Dimethylhexylen, Trimethylhexylen, Ethylhexylen, n- oder iso-Propylhexylen, Methylheptylen), einen $C_2$—$C_8$-Alkenylenrest (z.B. Vinylen, Allylen, Methylallylen, Butenylen, Methylbutenylen), einen durch Halogen substituierten $C_1$—$C_{12}$-Alkylenrest (z.B. Chlormethylen, Dichlormethylen, Fluormethylen, Difluormethylen, Brommethylen, Dibrommethylen, Chlorethylen, Fluorethylen, Bromethylen, Fluorpropylen, Chlorpropylen, Brompropylen, Fluorbutylen, Chlorbutylen, Brombutylen), einen durch Halogen substituierten $C_2$—$C_4$-Alkenylenrest (z.B. Chlorvinylen, Dichlorvinylen), einen durch Hydroxy substituierten $C_1$—$C_8$-Alkylenrest (z.B. Hydroxymethylen, Hydroxyethylen).

$X_n$ bedeutet für den Fall n = 0 eine Einfachbindung.

Die neuen Verbindungen der Formel I können bei ihrer Herstellung aufgrund der C = N-Doppelbindung als E/Z-Isomerengemische anfallen, die in üblicher Weise, z.B. durch kristallisation oder Chromatographie, in die einzelnen Komponenten getrennt werden können. Sowohl die einzelnen isomeren Verbindugen als auch ihre Gemisch werden von der Erfindung umfaßt und sind als Fungizide brauchbar.

Die neuen Verbindungen der Formel I können z.B. hergestellt werden, indem man ein orthosubstituiertes Benzylbromid der allgemeinen Formel III, in der $R^1$ und $R^2$ die oben angegebene Bedeutung haben, mit einem Alkali-, Erdalkali- oder Ammoniumsalz einer Carbonsäure der Formel II, worin $R^3$, X und n die oben angegebene Bedeutung haben, in einem Lösungs- oder Verdünnungsmittel und gegebenenfalls unter Zusatz eines Katalysators zu den neuen Verbindungen umsetzt.

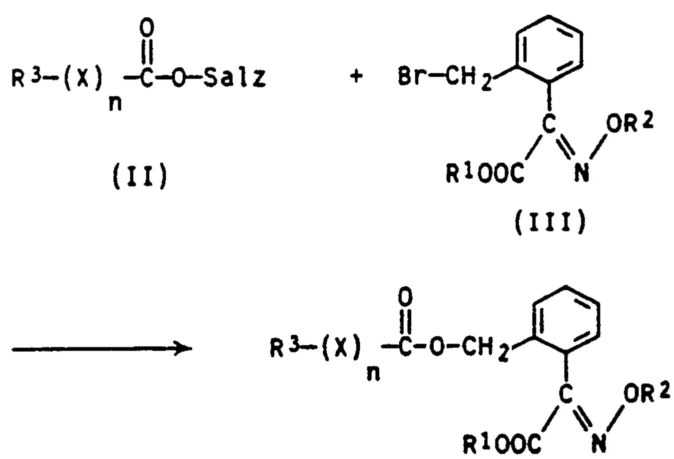

(II) (III)

(I)

Die Herstellung von Carbonsäureestern aus Alkylhalogeniden und Carboxylaten ist bekannt (vgl. z.B. Synthesis 1975, 805).

Als Lösungs- oder Verdünnungsmittel für die Reaktion von II mit III kommen Aceton, Acetonitril, Dimethylsulfoxid, Dioxan, Dimethylformamid, N-Methylpyrrolidon, N,N'-Dimethylpropylenharnstoff oder Pyridin in Betracht.

Außerdem kann es von Vorteil sein, der Reaktionsmischung einen Katalysator, wie z.B. Kaliumjodid oder Tetramethylethylendiamin, in einer Menge von 0,01 bis 10% (Gew.%), bezogen auf Verbindung III, zuzusetzen.

Die entsprechenden Umsetzungen können auch im Zweiphasensystem (z.B. Tetrachlorkohlenstoff/Wasser) durchgeführt werden. Als Phasentransferkatalysatoren kommen z.B. Trioctylpropylammoniumchlorid oder Cetyltrimethylammoniumchlorid in Betracht (vgl. Synthesis 1974, 867).

Die Carboxylate der Formel II sind bekannt. Sie können aus den entsprechenden Carbonsäuren mit Basen (z.B. Kaliumhydroxid) in einem inerten Lösungsmittel (z.B. Ethanol) hergestellt werden.

Die ortho-substituierten Benzylbromide der Formel III lassen sich herstellen, indem man literturbekannte α-Ketocarbonsäureester der Formel IV (vgl. z.B. J. M. Photis, Tetrahedron Lett. 1980, 3539)

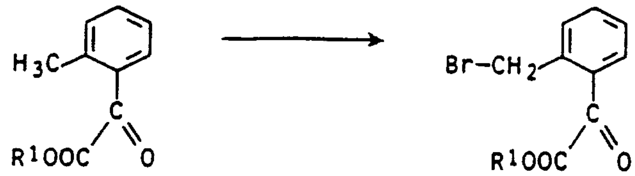

(IV) (V)

mit Brom in einem Lösungsmitel wie zum Beispiel Tetrachlormethan, gegebenenfalls unter Belichtung mit einer Lichtquelle (zum Beispiel Hg-Dampf-Lampe, 300 W), oder mit N-Bromsuccinimid (Horner, Winkelmann, Angew, Chem. 71, 349 (1959) zu den α-Ketocarbonsäureestern der allgemeinen Formel V umsetzt, wobei $R^1$ die obengenannten Bedeutungen hat.

Die Bromide der Formel III lassen sich herstellen, indem man einen α-Ketocarbonsäureester der Formel V a) mit O-substituierten Hydroxylaminen der Formel $H_2N\text{-}OR^2$ umsetzt, in der $R^2$ die obengenannten Bedeutungen hat, oder b) mit Hydroxylamin zum entsprechenden Oxim und danach mit einem Alkylhalogenid der Formel $R^2$-X, in der $R^2$ die obengenannten Bedeutungen hat und x ein Halogenatom (F, Cl, Br, J) bedeutet, oder mit einem Dialkylsulfat umsetzt.

Die neuen Verbindungen der Formel I können zum Beispiel auch in der Weise hergestellt werden, daß man die neuen α-Ketocarbonsäureester der Formel VI

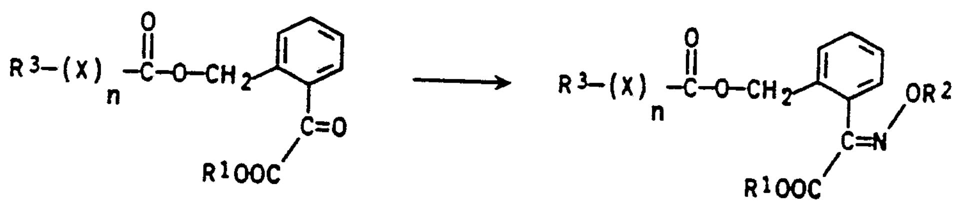

(VI) (I)

a) mit O-substituierten Hydroxylaminen der Formel $H_2NOR^2$ umsetzt, in der $R^2$ die obengenannten Bedeutungen hat, oder b) mit Hydroxylamin zum entsprechenden Oxim und danach mit einem Alkylhalogenid der Formel $R^2$-X, in der $R^2$ die obengenannten Bedeutungen hat und x ein Halogenatom (F, Cl, Br, J) bedeutet, oder mit einem Dialkylsulfat umsetzt.

Die neuen α-Ketocarbonsäureester der allgemeinen Formel VI sind wertvolle Zwischenprodukte. Sie können zum Beispiel hergestellt werden, indem man die obengenannte Verbindung der Formel V mit einem Alkali-, Erdalkali-oder Ammoniumsalz eine Carbonsäure der Formel II, worin $R^3$, $R^1$, X und n die obengenannten Bedeutungen haben, in einem Lösungs- oder Verdünnungsmittel und gegebenenfalls unter Zusatz eines Katalysators zu den neuen Verbindungen der Formel VI umsetzt:

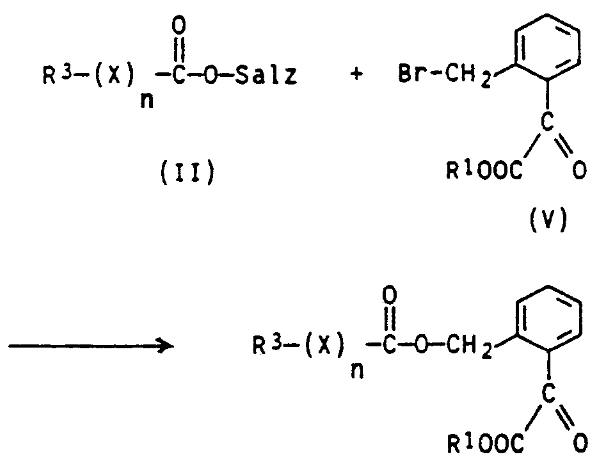

(II) (V)

(VI)

Die Herstellung von Carbonsäureestern aus Alkylhalogeniden und Carboxylaten ist bekannt (vlg. z.B. Synthesis 1975, 805).

Als Lösungs- oder Verdünnungsmittel für die Reaktion von II mit V kommen Aceton, Acetonitril, Dimethylsulfoxid, Dioxan, Dimethylformamid, N-Methylpyrrolidon, N,N'-Dimethylpropylenharnstoff oder Pyridin in Betracht.

Außerdem kann es von Vorteil sein, der Reaktionsmischung einen Katalysator, wie z.B. Kaliumjodid oder Tetramethylethylendiamin, in einer Menge von 0,01 bis 10% (Gew.%), bezogen auf Verbindung V zuzusetzen.

Die entsprechenden Umsetzungen können auch im Zweiphasensystem (z.B. Tetrachlorkohlenstoff/

Wasser) durchgeführt werden. Als Phasentransferkatalysatoren kommen z.B. Trioctylpropylammoniumchlorid oder Cetyltrimethylammoniumchlorid in Betracht (vgl. Synthesis 1974, 867).

Die Herstellung de neuen Verbindungen der Formel I wird durch folgende Beispiele erläutert.

Vorschrift 1

Herstellung von 2-(Brommethyl)-phenylglyoxylsäuremethylester

5,34 g (30 mmol) 2-Methylphenylglyoxylsäuremethylester und 5,34 g (30 mol) N-Bromsuccinimid werden in 1000 ml Tetrachlormethan für eine Stunde mit einer 300 W-Hg-Dampf-Lampe bestrahlt, Dan wird die organische Phase lx mit Wasser und 3x mit Natriumhydrogencarbonat-Lösung gewaschen und über Natriumsulfat/Natriumcarbonat getrocknet. Nach dem Einengen wird das Rohprodukt an Kieselgel mit Methyl-t.-Butylether/n-Hexan (1/9) chromatographiert. Man erhält 3,8 g (49%) der obengenannten Verbindungen als gelbes Öl.

$^1H$—NMR ($CDCl_3$): δ = 3,97 (S, 3H), 4,90 (S, 2H), 7,4—7.8 (m, 4H)

IR (Film): 2955, 1740, 1689, 1435, 1318, 1207, 999 $cm^{-1}$

Vorschrift 2

Herstellung von 2-(α-Methylcyclopropylcarboxymethylen)-phenylglyoxylsäuremethylester

13,8 g (0,1 mol) des Kaliumsalzes der α-Methylcyclopropancarbonsäure werden mit 21,1 g (0,082 mol) 2-(Brommethyl)-phenylglyoxysäuremethylester und 0,3 g Kaliumiodid in 300 ml N-Methylpyrrolidon gelöst. Man läßt 15 h bei 23°C rühren, gießt auf 300 m Eiswasser und extrahiert mit 3 × 200 ml Methyl-tert. Butylether. Die organischen Phasen werden mit Wasser gewaschen, mit Natriumsulfat getrocknet und eingeengt. Man erhält die oben genannte Verbindung in quantitativer Ausbeute.

$^1H$($CDCl_3$): δ = 0,70 (m, 2H), 1,27 (m, 2H), 1,35 (s, 3H), 3,96 (s, 3H), 5,46 (s, 2H), 7, 4—7,8 (m, 4H).

Vorschrift 3

Herstellung von 2-(Brommethyl)-phenylglyoxylsäuremethylester-O-methyloxim

27,5 g (0,133 mol) 2-Methyl-phenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampflampe vier Stunden auf Rückflußtemperatur erhitzt. Danach wird eingeengt, in Essigester/Wasser aufgenommen, mit $H_2O$ gewaschen, mit Natriumsulfat getrocknet und eingeengt. Das Rohprodukt wird mit Cyclohexan/Essigester (9/1) an Kieselgel chromatographisch gereinigt. Es werden 17,4 g (46%) der oben genannten Verbindung als Öl erhalten.

$^1H$($CDCl_3$): δ = 3,88 (s, 3H), 4,08 (s, 3H), 4,33 (s, 2H), 7.12—7,52 (m, 4H).

## Beispiel 1

2-(α-Methylcyclopropylcarboxymethylen)-phenylglyoxylsäuremethylester-O-methyloxim (Verbindung Nr. 316)

5,1 g (37 mmol) α-Methylcyclopropylcarbonsäure-Kaliumsalz werden zusammen mit einer Spatelspitze Kaliumjodid in 125 ml absoluten N,N-Dimethylformaid vorgelegt. Dazu tropft man 8,9 g (31 mmol) 2-(Brommethyl)-phenylglyoxylsäuremethylester-O-methyloxim (in wenig N,N-Dimethylformamid gelöst) und rührt 5 Stunden bei 100°C. Dann wird abgesauagt und eingeengt.

Der Rückstand wird in Ether aufgenommen, mit $H_2O$ gewaschen, über Natriumsulfat getrocknet und eingeengt. Man chromatographiert an Kieselgel mit Cyclohexan/Essigester (9/1) und erhält 2,7 g (28,5%) der obengenannten Verbindung als weiße Kristalle. Fp = 94—96°C.

$^1H$—NMR($CDCl_3$): δ = 0,65 (m, 2H), 1,20 (m, 2H), 1,30 (s, 3H), 3,85 (s, 3h), 4,05 (s, 3H), 4,95 s, 2H), 7,18 (m, 1H), 7,40 (m, 3H).

In entsprechender Weise können die in der nachfolgenden Tabelle aufgeführten Verbindungen hergestellt werden.

Tabelle 1

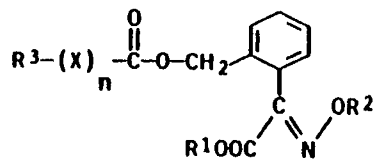

| Nr. | $R^3$ | $(X)_n$ | $R^2$ | $R^1$ | Fp (°C) | IR ($cm^{-1}$) |
|---|---|---|---|---|---|---|
| 1 | H | $-CH_2-$ | $CH_3$ | $CH_3$ | | |
| 2 | H | $-CH_2-CH_2-$ | $CH_3$ | $CH_3$ | | |
| 3 | H | $-CH_2-CH(CH_3)-$ | $CH_3$ | $CH_3$ | | |
| 4 | H | $-CH_2-C(CH_3)_2-$ | $CH_3$ | $CH_3$ | 56-59 | 2980, 1728, 1281, 1221, 1159, 1069, 1020, 959 |
| 5 | H | $-CH=CH-$ | $CH_3$ | $CH_3$ | | |
| 6 | H | $-CH=C(CH_3)-$ | $CH_3$ | $CH_3$ | | |
| 7 | H | $-C\equiv C-$ | $CH_3$ | $CH_3$ | | |
| 8 | H | $-CH_2-CH_2-CH_2-$ | $CH_3$ | $CH_3$ | | |
| 9 | H | $-CH_2-CH_2-CH(CH_3)-$ | $CH_3$ | $CH_3$ | | |
| 10 | H | $-CH_2-CH(CH_3)-CH_2-$ | $CH_3$ | $CH_3$ | | |
| 11 | H | $-CH_2-CH_2-C(CH_3)_2-$ | $CH_3$ | $CH_3$ | 44-47 | 2980, 1738, 1719, 1297, 1155, 1067, 1008, 772 |
| 12 | H | $-CH_2-C(CH_3)_2-CH_2-$ | $CH_3$ | $CH_3$ | | |
| 13 | H | $-CH_2-CH_2-C(C_2H_5)_2-$ | $CH_3$ | $CH_3$ | | |
| 14 | H | $-CH=CH-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 15 | H | $-CH_2-CH=CH-$ | $CH_3$ | $CH_3$ | | |
| 16 | H | $-CH_2-C(CH_3)=CH-$ | $CH_3$ | $CH_3$ | | |
| 17 | H | $-CH_2-CH=C(CH_3)-$ | $CH_3$ | $CH_3$ | | |
| 18 | H | $-(CH_2)_4-$ | $CH_3$ | $CH_3$ | | |
| 19 | H | $-CH_2-CH_2-CH_2-CH(CH_3)-$ | $CH_3$ | $CH_3$ | | |
| 20 | H | $-CH_2-CH(CH_3)-CH_2-CH_2-$ | $CH_3$ | $CH_3$ | | |
| 21 | H | $-(CH_2)_3-C(CH_3)_2-$ | $CH_3$ | $CH_3$ | | |
| 22 | H | $-(CH_2)_3-CH(C_2H_5)-$ | $CH_3$ | $CH_3$ | öl | 2958, 1732, 1220, 1069, 1020 |
| 23 | H | $-(CH_2)_3-CH(n-C_3H_7)-$ | $CH_3$ | $CH_3$ | | |
| 24 | H | $-CH_2-CH=CH-CH_2-$ | $CH_3$ | $CH_3$ | | |
| 25 | H | $-CH_2-C(CH_3)=CH-CH_2-$ | $CH_3$ | $CH_3$ | | |
| 26 | H | $-(CH_2)_5-$ | $CH_3$ | $CH_3$ | | |
| 27 | H | $-(CH_2)_4-CH(CH_3)-$ | $CH_3$ | $CH_3$ | | |
| 28 | H | $-(CH_2)_4-CH(C_2H_5)-$ | $CH_3$ | $CH_3$ | | |
| 29 | H | $-(CH_2)_3-CH(CH_3)-CH_2-$ | $CH_3$ | $CH_3$ | | |
| 30 | H | $-CH_2-CH=CH-CH=CH-$ | $CH_3$ | $CH_3$ | | |
| 31 | H | $-CH_2-C(CH_3)=CH-CH=CH-$ | $CH_3$ | $CH_3$ | öl | 2935, 1737, 1221, 1069, 1020 |
| 32 | H | $-(CH_2)_6-$ | $CH_3$ | $CH_3$ | | |
| 33 | H | $-(CH_2)_5-CH(CH_3)-$ | $CH_3$ | $CH_3$ | | |
| 34 | H | $-(CH_2)_4-CH(CH_3)-CH_2-$ | $CH_3$ | $CH_3$ | | |
| 35 | H | $-(CH_2)_5-CH(n-C_3H_7)-$ | $CH_3$ | $CH_3$ | | |
| 36 | H | $-(CH_2)_7-$ | $CH_3$ | $CH_3$ | | |
| 37 | H | $-(CH_2)_6-CH(CH_3)-$ | $CH_3$ | $CH_3$ | | |
| 38 | H | $-(CH_2)_5-CH(CH_3)-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 39 | H | $-(CH_2)_6-C(CH_3)_2-$ | $CH_3$ | $CH_3$ | | |
| 40 | H | $-(CH_2)_8-$ | $CH_3$ | $CH_3$ | | |
| 41 | H | $-(CH_2)_9-$ | $CH_3$ | $CH_3$ | | |
| 42 | H | $-(CH_2)_{10}-$ | $CH_3$ | $CH_3$ | | |
| 43 | H | $-CHCl-$ | $CH_3$ | $CH_3$ | | |
| 44 | H | $-CCl_2-$ | $CH_3$ | $CH_3$ | | |
| 45 | Cl | $-CCl_2-$ | $CH_3$ | $CH_3$ | | |
| 46 | H | $-CHBr-$ | $CH_3$ | $CH_3$ | | |
| 47 | H | $-CBr_2-$ | $CH_3$ | $CH_3$ | | |
| 48 | Br | $-CBr_2$ | $CH_3$ | $CH_3$ | | |
| 49 | H | $-CHF-$ | $CH_3$ | $CH_3$ | | |
| 50 | H | $-CF_2-$ | $CH_3$ | $CH_3$ | | |
| 51 | F | $-CF_2-$ | $CH_3$ | $CH_3$ | | |
| 52 | H | $-CH=CCl-$ | $CH_3$ | $CH_3$ | | |
| 53 | H | $-CCl=CCl-$ | $CH_3$ | $CH_3$ | | |
| 54 | Cl | $-C(CH_3)_2-$ | $CH_3$ | $CH_3$ | | |
| 55 | H | $-C(CH_3)_2-$ | $CH_3$ | $CH_3$ | | |
| 56 | H | $-CHCl-CH(CH_3)-$ | $CH_3$ | $CH_3$ | | |
| 57 | H | $-CHCl-C(CH_3)_2-$ | $CH_3$ | $CH_3$ | | |
| 58 | H | $-CHBr-CH(CH_3)-$ | $CH_3$ | $CH_3$ | | |
| 59 | Br | $-C(C_2H_5)_2-$ | $CH_3$ | $CH_3$ | | |
| 60 | H | $-CH(OH)-$ | $CH_3$ | $CH_3$ | | |
| 61 | H | $-CH_2-CH(OH)-$ | $CH_3$ | $CH_3$ | | |
| 62 | H | $-CH_2-CH_2-CH(OH)-$ | $CH_3$ | $CH_3$ | | |
| 63 | H | $-CH_2-CH(OH)-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 64 | H | $-CH(OH)-CH_2-$ | $CH_3$ | $CH_3$ | | |
| 65 | H | $-CH(OH)-C(CH_3)_2-$ | $CH_3$ | $CH_3$ | | |
| 66 | H | $-CH_2-C(OH)(CH_3)-$ | $CH_3$ | $CH_3$ | | |
| 67 | H | $-CH_2-CH(CH_3)-CH(OH)-$ | $CH_3$ | $CH_3$ | | |
| 68 | H | $-CH=CH-CH(OH)-$ | $CH_3$ | $CH_3$ | | |
| 69 | H | $-CH=CH-CH_2-CH(OH)-$ | $CH_3$ | $CH_3$ | | |
| 70 | CN | $-CH_2-$ | $CH_3$ | $CH_3$ | 68-70 | 2950, 1729, 398, 1222, 1167, 1069 1019 |
| 71 | Cyclopropyl | - | $CH_3$ | $CH_3$ | | |
| 72 | Cyclobutyl | - | $CH_3$ | $CH_3$ | | |
| 73 | Cyclopentyl | - | $CH_3$ | $CH_3$ | | |
| 74 | Cyclohexyl | - | $CH_3$ | $CH_3$ | | |
| 75 | Adamantyl | - | $CH_3$ | $CH_3$ | | |
| 76 | 9-Fluorenyl | - | $CH_3$ | $CH_3$ | | |
| 77 | Cyclopentyl | $-CH_2-$ | $CH_3$ | $CH_3$ | | |
| 78 | 3-Cyclopentenyl | $-CH_2-$ | $CH_3$ | $CH_3$ | | |
| 79 | Cyclohexyl | $-CH_2-$ | $CH_3$ | $CH_3$ | | |
| 80 | Cyclopentyl | $-CH_2-CH_2-$ | $CH_3$ | $CH_3$ | | |
| 81 | Cyclohexyl | $-CH_2-CH_2-$ | $CH_3$ | $CH_3$ | | |
| 82 | Cyclohexyl | $-(CH_2)_3-$ | $CH_3$ | $CH_3$ | | |
| 83 | $C_6H_5$ (=Phenyl) | - | $CH_3$ | $CH_3$ | | |
| 84 | $2-CH_3-C_6H_4$ | - | $CH_3$ | $CH_3$ | | |
| 85 | $3-CH_3-C_6H_4$ | - | $CH_3$ | $CH_3$ | | |
| 86 | $4-CH_3-C_6H_4$ | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 87 | $2,3-(CH_3)_2-C_6H_3$ | - | $CH_3$ | $CH_3$ | | |
| 88 | $2,4-(CH_3)_2-C_6H_3$ | - | $CH_3$ | $CH_3$ | | |
| 89 | $2,6-(CH_3)_2-C_6H_3$ | - | $CH_3$ | $CH_3$ | 84-86 | 2960, 1726, 1261, 1245, 1069, 1014, 784 |
| 90 | $3,4-(CH_3)_2-C_6H_3$ | - | $CH_3$ | $CH_3$ | | |
| 91 | $3,5-(CH_3)_2-C_6H_3$ | - | $CH_3$ | $CH_3$ | | |
| 92 | $2,4,6-(CH_3)_3-C_6H_2$ | - | $CH_3$ | $CH_3$ | Öl | 2950, 1727, 1437, 1262, 1071, 1019 |
| 93 | $4-t-C_4H_9-C_6H_4$ | - | $CH_3$ | $CH_3$ | | |
| 94 | $2-C_6H_5-C_6H_4$ | - | $CH_3$ | $CH_3$ | | |
| 95 | $4-C_6H_5-C_6H_4$ | - | $CH_3$ | $CH_3$ | | |
| 96 | 2-Benzyl-$C_6H_4$ | - | $CH_3$ | $CH_3$ | | |
| 97 | 4-Benzyl-$C_6H_4$ | - | $CH_3$ | $CH_3$ | | |
| 98 | $2-Cl-C_6H_4$ | - | $CH_3$ | $CH_3$ | | |
| 99 | $3-Cl-C_6H_4$ | - | $CH_3$ | $CH_3$ | | |
| 100 | $4-Cl-C_6H_4$ | - | $CH_3$ | $CH_3$ | | |
| 101 | $2,4-Cl_2-C_6H_3$ | - | $CH_3$ | $CH_3$ | | |
| 102 | $2,5-Cl_2-C_6H_3$ | - | $CH_3$ | $CH_3$ | | |
| 103 | $2,6-Cl_2-C_6H_3$ | - | $CH_3$ | $CH_3$ | 73-75 | 2950, 1738, 1433, 1270, 1142, 1069, 1019 |
| 104 | $3,4-Cl_2-C_6H_3$ | - | $CH_3$ | $CH_3$ | | |
| 105 | $3,5-Cl_2-C_6H_3$ | - | $CH_3$ | $CH_3$ | | |
| 106 | $2,4,5-Cl_3-C_6H_2$ | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 107 | 2,3,4,5,6-$Cl_5$-$C_6$ | - | $CH_3$ | $CH_3$ | | |
| 108 | 2-F,4-Cl-$C_6H_3$ | - | $CH_3$ | $CH_3$ | | |
| 109 | 2-F-$C_6H_4$ | - | $CH_3$ | $CH_3$ | | |
| 110 | 3-F-$C_6H_4$ | - | $CH_3$ | $CH_3$ | | |
| 111 | 4-F-$C_6H_4$ | - | $CH_3$ | $CH_3$ | | |
| 112 | 2,4-$F_2$-$C_6H_3$ | - | $CH_3$ | $CH_3$ | | |
| 113 | 2,6-$F_2$-$C_6H_3$ | - | $CH_3$ | $CH_3$ | Öl | 2950, 1734, 1625, 1470, 1287, 1263, 1069, 1016 |
| 114 | 2,3,4,5,6-$F_5$-$C_6$ | - | $CH_3$ | $CH_3$ | | |
| 115 | 2-$CF_3$-$C_6H_4$ | - | $CH_3$ | $CH_3$ | | |
| 116 | 3-$CF_3$-$C_6H_4$ | - | $CH_3$ | $CH_3$ | | |
| 117 | 4-$CF_3$-$C_6H_4$ | - | $CH_3$ | $CH_3$ | | |
| 118 | 2-$OCH_3$-$C_6H_4$ | - | $CH_3$ | $CH_3$ | | |
| 119 | 3-$OCH_3$-$C_6H_4$ | - | $CH_3$ | $CH_3$ | | |
| 120 | 4-$OCH_3$-$C_6H_4$ | - | $CH_3$ | $CH_3$ | | |
| 121 | 2-Phenoxy-$C_6H_4$ | - | $CH_3$ | $CH_3$ | | |
| 122 | 3-Phenoxy-$C_6H_4$ | - | $CH_3$ | $CH_3$ | | |
| 123 | 4-Phenoxy-$C_6H_4$ | - | $CH_3$ | $CH_3$ | | |
| 124 | 4-Ethoxy-$C_6H_4$ | - | $CH_3$ | $CH_3$ | | |
| 125 | 2-Phenoxyethoxy-$C_6H_4$ | - | $CH_3$ | $CH_3$ | | |
| 126 | 2-(2'-Cl-Phenoxyethoxy)-$C_6H_4$ | - | $CH_3$ | $CH_3$ | | |
| 127 | 2-(3'-Cl-Phenoxyethoxy)-$C_6H_4$ | - | $CH_3$ | $CH_3$ | | |
| 128 | 2-(4'-Cl-Phenoxyethoxy)-$C_6H_4$ | - | $CH_3$ | $CH_3$ | | |
| 129 | 3-Phenoxyethoxy-$C_6H_4$ | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 130 | 3-(4'-Cl-Phenoxyethoxy)-$C_6H_4$ | - | $CH_3$ | $CH_3$ | | |
| 131 | 4-Phenoxyethoxy-$C_6H_4$ | - | $CH_3$ | $CH_3$ | | |
| 132 | 2-Phenoxypropoxy-$C_6H_4$ | - | $CH_3$ | $CH_3$ | | |
| 133 | 3-Phenoxypropoxy-$C_6H_4$ | - | $CH_3$ | $CH_3$ | | |
| 134 | 4-Phenoxypropoxy-$C_6H_4$ | - | $CH_3$ | $CH_3$ | | |
| 135 | $C_6H_5$ | $-CH_2-$ | $CH_3$ | $CH_3$ | | |
| 136 | 2-$CH_3$-$C_6H_4$ | $-CH_2-$ | $CH_3$ | $CH_3$ | | |
| 137 | $C_6H_5$ | $-CH(CH_3)-$ | $CH_3$ | $CH_3$ | | |
| 138 | 4-Phenyl-$C_6H_4$ | $-CH_2-$ | $CH_3$ | $CH_3$ | | |
| 139 | 2-F-$C_6H_4$ | $-CH_2-$ | $CH_3$ | $CH_3$ | | |
| 140 | 3-F-$C_6H_4$ | $-CH_2-$ | $CH_3$ | $CH_3$ | | |
| 141 | 4-F-$C_6H_4$ | $-CH_2-$ | $CH_3$ | $CH_3$ | | |
| 142 | 2-Cl-$C_6H_4$ | $-CH_2-$ | $CH_3$ | $CH_3$ | | |
| 143 | 3-Cl-$C_6H_4$ | $-CH_2-$ | $CH_3$ | $CH_3$ | | |
| 144 | 4-Cl-$C_6H_4$ | $-CH_2-$ | $CH_3$ | $CH_3$ | Öl | 2950, 1737, 1492, 1231, 1221, 1070, 1016 |
| 145 | 2,4-$Cl_2$-$C_6H_3$ | $-CH_2-$ | $CH_3$ | $CH_3$ | | |
| 146 | 2,6-$Cl_2$-$C_6H_3$ | $-CH_2-$ | $CH_3$ | $CH_3$ | | |
| 147 | 2-Cl,4-F-$C_6H_3$ | $-CH_2-$ | $CH_3$ | $CH_3$ | | |
| 148 | 2-Ethoxy-$C_6H_4$ | $-CH_2-$ | $CH_3$ | $CH_3$ | | |
| 149 | 4-Ethoxy-$C_6H_4$ | $-CH_2-$ | $CH_3$ | $CH_3$ | | |
| 150 | 2-$OCH_3$-$C_6H_4$ | $-CH_2-$ | $CH_3$ | $CH_3$ | | |
| 151 | 4-$OCH_3$-$C_6H_4$ | $-CH_2-$ | $CH_3$ | $CH_3$ | | |
| 152 | 4-t-$C_4H_9$-$C_6H_4$ | $-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 153 | $C_6H_5$ | $-CH(iso-C_3H_7)-$ | $CH_3$ | $CH_3$ | | |
| 154 | $4-Cl-C_6H_4$ | $-CH(iso-C_3H_7)-$ | $CH_3$ | $CH_3$ | | |
| 155 | $4-F-C_6H_4$ | $-CH(iso-C_3H_7)-$ | $CH_3$ | $CH_3$ | | |
| 156 | $4-OCF_2H-C_6H_4$ | $-CH(iso-C_3H_7)-$ | $CH_3$ | $CH_3$ | | |
| 157 | $C_6H_5$ | $-CH(OH)-$ | $CH_3$ | $CH_3$ | | |
| 158 | $2-OCH_3-C_6H_4$ | $-CH(OH)-$ | $CH_3$ | $CH_3$ | | |
| 159 | $3-OCH_3-C_6H_4$ | $-CH(OH)-$ | $CH_3$ | $CH_3$ | | |
| 160 | $4-OCH_3-C_6H_4$ | $-CH(OH)-$ | $CH_3$ | $CH_3$ | | |
| 161 | $4-Cl-C_6H_4$ | $-CH(OH)-$ | $CH_3$ | $CH_3$ | | |
| 162 | $C_6H_5$ | $-CH(CH_2OH)-$ | $CH_3$ | $CH_3$ | | |
| 163 | $C_6H_5$ | $-CH_2-CH_2-$ | $CH_3$ | $CH_3$ | | |
| 164 | $C_6H_5$ | $-CH(CH_3)-CH_2-$ | $CH_3$ | $CH_3$ | | |
| 165 | $C_6H_5$ | $-CH_2-CH(CH_3)-$ | $CH_3$ | $CH_3$ | | |
| 166 | $C_6H_5$ | $-CH(CH_3)-CH(CH_3)-$ | $CH_3$ | $CH_3$ | | |
| 167 | $C_6H_5$ | $-CH(C_6H_5)-CH_2-$ | $CH_3$ | $CH_3$ | | |
| 168 | $4-t-C_4H_9-C_6H_4$ | $-CH_2-CH_2-$ | $CH_3$ | $CH_3$ | | |
| 169 | $4-t-C_4H_9-C_6H_4$ | $-CH_2-CH(CH_3)-$ | $CH_3$ | $CH_3$ | | |
| 170 | $2-Cl-C_6H_4$ | $-CH_2-CH_2-$ | $CH_3$ | $CH_3$ | | |
| 171 | $3-Cl-C_6H_4$ | $-CH_2-CH_2-$ | $CH_3$ | $CH_3$ | | |
| 172 | $4-Cl-C_6H_4$ | $-CH_2-CH_2-$ | $CH_3$ | $CH_3$ | | |
| 173 | $2-F-C_6H_4$ | $-CH_2-CH_2-$ | $CH_3$ | $CH_3$ | | |
| 174 | $3-F-C_6H_4$ | $-CH_2-CH_2-$ | $CH_3$ | $CH_3$ | | |
| 175 | $4-F-C_6H_4$ | $-CH_2-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 176 | $2-OCH_3-C_6H_4$ | $-CH_2-CH_2-$ | $CH_3$ | $CH_3$ | | |
| 177 | $4-OCH_3-C_6H_4$ | $-CH_2-CH_2-$ | $CH_3$ | $CH_3$ | | |
| 178 | $C_6H_5$ | -CH=CH- | $CH_3$ | $CH_3$ | | |
| 179 | $2-Cl-C_6H_4$ | -CH=CH- | $CH_3$ | $CH_3$ | | |
| 180 | $3-Cl-C_6H_4$ | -CH=CH- | $CH_3$ | $CH_3$ | | |
| 181 | $4-Cl-C_6H_4$ | -CH=CH- | $CH_3$ | $CH_3$ | | |
| 182 | $2,6-Cl_2-C_6H_3$ | -CH=CH- | $CH_3$ | $CH_3$ | | |
| 183 | $2,4-Cl_2-C_6H_3$ | -CH=CH- | $CH_3$ | $CH_3$ | | |
| 184 | $2-F-C_6H_4$ | -CH=CH- | $CH_3$ | $CH_3$ | | |
| 185 | $3-F-C_6H_4$ | -CH=CH- | $CH_3$ | $CH_3$ | | |
| 186 | $4-F-C_6H_4$ | -CH=CH- | $CH_3$ | $CH_3$ | | |
| 187 | $2-CF_3-C_6H_4$ | -CH=CH- | $CH_3$ | $CH_3$ | | |
| 188 | $4-CF_3-C_6H_4$ | -CH=CH- | $CH_3$ | $CH_3$ | | |
| 189 | $2-CH_3-C_6H_4$ | -CH=CH- | $CH_3$ | $CH_3$ | | |
| 190 | $4-CH_3-C_6H_4$ | -CH=CH- | $CH_3$ | $CH_3$ | | |
| 191 | $4-i-C_3H_7-C_6H_4$ | -CH=CH- | $CH_3$ | $CH_3$ | | |
| 192 | $4-t-C_4H_9-C_6H_4$ | -CH=CH- | $CH_3$ | $CH_3$ | | |
| 193 | $2-OCH_3-C_6H_4$ | -CH=CH- | $CH_3$ | $CH_3$ | | |
| 194 | $3-OCH_3-C_6H_4$ | -CH=CH- | $CH_3$ | $CH_3$ | | |
| 195 | $4-OCH_3-C_6H_4$ | -CH=CH- | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 196 | 2-Phenoxy-$C_6H_4$ | $-CH=CH-$ | $CH_3$ | $CH_3$ | | |
| 197 | 3-Phenoxy-$C_6H_4$ | $-CH=CH-$ | $CH_3$ | $CH_3$ | | |
| 198 | 4-Phenoxy-$C_6H_4$ | $-CH=CH-$ | $CH_3$ | $CH_3$ | | |
| 199 | $C_6H_5$ | $-(CH_2)_3-$ | $CH_3$ | $CH_3$ | | |
| 200 | $C_6H_5$ | $-CH(CH_3)-CH_2-CH_2-$ | $CH_3$ | $CH_3$ | | |
| 201 | $C_6H_5$ | $-CH_2-CH(CH_3)-CH_2-$ | $CH_3$ | $CH_3$ | | |
| 202 | $C_6H_5$ | $-CH_2-CH_2-CH(CH_3)-$ | $CH_3$ | $CH_3$ | | |
| 203 | 2-Cl-$C_6H_4$ | $-(CH_2)_3-$ | $CH_3$ | $CH_3$ | | |
| 204 | 4-Cl-$C_6H_4$ | $-(CH_2)_3-$ | $CH_3$ | $CH_3$ | | |
| 205 | 2-$OCH_3$-$C_6H_4$ | $-(CH_2)_3-$ | $CH_3$ | $CH_3$ | | |
| 206 | 4-$OCH_3$-$C_6H_4$ | $-(CH_2)_3-$ | $CH_3$ | $CH_3$ | | |
| 207 | 4-t-$C_4H_9$-$C_6H_4$ | $-(CH_2)_3-$ | $CH_3$ | $CH_3$ | | |
| 208 | $C_6H_5$ | $-CH=CH-CH_2-$ | $CH_3$ | $CH_3$ | | |
| 209 | $C_6H_5$ | $-(CH_2)_4-$ | $CH_3$ | $CH_3$ | | |
| 210 | 2-Cl-$C_6H_4$ | $-(CH_2)_4-$ | $CH_3$ | $CH_3$ | | |
| 211 | 4-Cl-$C_6H_4$ | $-(CH_2)_4-$ | $CH_3$ | $CH_3$ | | |
| 212 | 2-$OCH_3$-$C_6H_4$ | $-(CH_2)_4-$ | $CH_3$ | $CH_3$ | | |
| 213 | 4-$OCH_3$-$C_6H_4$ | $-(CH_2)_4-$ | $CH_3$ | $CH_3$ | | |
| 214 | 4-$CF_3$-$C_6H_4$ | $-(CH_2)_4-$ | $CH_3$ | $CH_3$ | | |
| 215 | 2-$CH_3$-$C_6H_4$ | $-(CH_2)_4-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 216 | $4-CH_3-C_6H_4$ | $-(CH_2)_4-$ | $CH_3$ | $CH_3$ | | |
| 217 | $C_6H_5$ | $-CH_2-CH_2-CH(CH_3)-CH_2-$ | $CH_3$ | $CH_3$ | | |
| 218 | $C_6H_5$ | $-(CH_2)_5-$ | $CH_3$ | $CH_3$ | | |
| 219 | $2-CH_3-C_6H_4$ | $-(CH_2)_5-$ | $CH_3$ | $CH_3$ | | |
| 220 | $4-CH_3-C_6H_4$ | $-(CH_2)_5-$ | $CH_3$ | $CH_3$ | | |
| 221 | $2-Cl-C_6H_4$ | $-(CH_2)_5-$ | $CH_3$ | $CH_3$ | | |
| 222 | $4-Cl-C_6H_4$ | $-(CH_2)_5-$ | $CH_3$ | $CH_3$ | | |
| 223 | $2-OCH_3-C_6H_4$ | $-(CH_2)_5-$ | $CH_3$ | $CH_3$ | | |
| 224 | $4-OCH_3-C_6H_4$ | $-(CH_2)_5-$ | $CH_3$ | $CH_3$ | | |
| 225 | $4-CF_3-C_6H_4$ | $-(CH_2)_5-$ | $CH_3$ | $CH_3$ | | |
| 226 | $C_6H_5$ | $-CH_2-CH_2-CH_2-CH(CH_3)-CH_2-$ | $CH_3$ | $CH_3$ | | |
| 227 | $C_6H_5$ | $-CH_2-CH(CH_3)-CH_2-CH(CH_3)-CH_2-$ | $CH_3$ | $CH_3$ | | |
| 228 | $4-t-C_4H_9-C_6H_4$ | $-CH_2-CH(CH_3)-CH_2-CH(CH_3)-CH_2-$ | $CH_3$ | $CH_3$ | | |
| 229 | $C_6H_5$ | $-(CH_2)_6-$ | $CH_3$ | $CH_3$ | | |
| 230 | $C_6H_5$ | $-(CH_2)_4-CH(CH_3)-CH_2-$ | $CH_3$ | $CH_3$ | | |
| 231 | $C_6H_5-O-$ | $-CH_2-$ | $CH_3$ | $CH_3$ | | |
| 232 | $2-Cl-C_6H_4-O-$ | $-CH_2-$ | $CH_3$ | $CH_3$ | | |
| 233 | $3-Cl-C_6H_4-O-$ | $-CH_2-$ | $CH_3$ | $CH_3$ | | |
| 234 | $4-Cl-C_6H_4-O-$ | $-CH_2-$ | $CH_3$ | $CH_3$ | | |
| 235 | $2,4-Cl_2-C_6H_3-O-$ | $-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 236 | $2-CH_3-C_6H_4-O-$ | $-CH_2-$ | $CH_3$ | $CH_3$ | | |
| 237 | $4-CH_3-C_6H_4-O-$ | $-CH_2-$ | $CH_3$ | $CH_3$ | | |
| 238 | $2-OCH_3-C_6H_4-O-$ | $-CH_2-$ | $CH_3$ | $CH_3$ | | |
| 239 | $4-OCH_3-C_6H_4-O-$ | $-CH_2-$ | $CH_3$ | $CH_3$ | | |
| 240 | $4-CF_3-C_6H_4-O-$ | $-CH_2-$ | $CH_3$ | $CH_3$ | | |
| 241 | $C_6H_5-O-$ | $-CH(CH_3)-$ | $CH_3$ | $CH_3$ | | |
| 242 | $C_6H_5-O-$ | $-CH_2-CH_2-$ | $CH_3$ | $CH_3$ | | |
| 243 | $2-Cl-C_6H_4-O-$ | $-CH_2-CH_2-$ | $CH_3$ | $CH_3$ | | |
| 244 | $4-Cl-C_6H_4-O-$ | $-CH_2-CH_2-$ | $CH_3$ | $CH_3$ | | |
| 245 | $2-CH_3-C_6H_4-O-$ | $-CH_2-CH_2-$ | $CH_3$ | $CH_3$ | | |
| 246 | $4-CH_3-C_6H_4-O-$ | $-CH_2-CH_2-$ | $CH_3$ | $CH_3$ | | |
| 247 | $2-OCH_3-C_6H_4-O-$ | $-CH_2-CH_2-$ | $CH_3$ | $CH_3$ | | |
| 248 | $4-OCH_3-C_6H_4-O-$ | $-CH_2-CH_2-$ | $CH_3$ | $CH_3$ | | |
| 249 | $4-t-C_4H_9-C_6H_4-O-$ | $-CH_2-CH_2-$ | $CH_3$ | $CH_3$ | | |
| 250 | $4-sec.-C_4H_9-C_6H_4-O-$ | $-CH_2-CH_2-$ | $CH_3$ | $CH_3$ | | |
| 251 | $C_6H_5-O-$ | $-(CH_2)_3-$ | $CH_3$ | $CH_3$ | | |
| 252 | $2-Cl-C_6H_4-O-$ | $-(CH_2)_3-$ | $CH_3$ | $CH_3$ | | |
| 253 | $4-Cl-C_6H_4-O-$ | $-(CH_2)_3-$ | $CH_3$ | $CH_3$ | | |
| 254 | $3-F-C_6H_4-O-$ | $-(CH_2)_3-$ | $CH_3$ | $CH_3$ | | |
| 255 | $4-F-C_6H_4-O-$ | $-(CH_2)_3-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 256 | $2-CH_3-C_6H_4-O-$ | $-(CH_2)_3-$ | $CH_3$ | $CH_3$ | | |
| 257 | $4-CH_3-C_6H_4-O-$ | $-(CH_2)_3-$ | $CH_3$ | $CH_3$ | | |
| 258 | $2-OCH_3-C_6H_4-O-$ | $-(CH_2)_3-$ | $CH_3$ | $CH_3$ | | |
| 259 | $4-OCH_3-C_6H_4-O-$ | $-(CH_2)_3-$ | $CH_3$ | $CH_3$ | | |
| 260 | $2,4-Cl_2-C_6H_3-O-$ | $-(CH_2)_3-$ | $CH_3$ | $CH_3$ | | |
| 261 | $4-Cl-C_6H_4-O-$ | $-CH(CH_3)-CH_2-CH_2-$ | $CH_3$ | $CH_3$ | | |
| 262 | $2-CF_3-C_6H_4-O-$ | $-(CH_2)_3-$ | $CH_3$ | $CH_3$ | | |
| 263 | $3-CF_3-C_6H_4-O-$ | $-(CH_2)_3-$ | $CH_3$ | $CH_3$ | | |
| 264 | $4-CF_3-C_6H_4-O-$ | $-(CH_2)_3-$ | $CH_3$ | $CH_3$ | | |
| 265 | $4-t-Butoxy-C_6H_4-O-$ | $-(CH_2)_3-$ | $CH_3$ | $CH_3$ | | |
| 266 | $2-CH_3,4-Cl-C_6H_3-O-$ | $-(CH_2)_3-$ | $CH_3$ | $CH_3$ | | |
| 267 | $4-C_2H_5-C_6H_4-O-$ | $-(CH_2)_3-$ | $CH_3$ | $CH_3$ | | |
| 268 | $4-iso-C_3H_7-C_6H_4-O-$ | $-(CH_2)_3-$ | $CH_3$ | $CH_3$ | | |
| 269 | $4-t-C_4H_9-C_6H_4-O-$ | $-(CH_2)_3-$ | $CH_3$ | $CH_3$ | | |
| 270 | $C_6H_5-O-$ | $-CH_2-CH(CH_3)-CH_2-$ | $CH_3$ | $CH_3$ | | |
| 271 | $C_6H_5-O-$ | $-(CH_2)_4-$ | $CH_3$ | $CH_3$ | | |
| 272 | $2-Cl-C_6H_4-O-$ | $-(CH_2)_4-$ | $CH_3$ | $CH_3$ | | |
| 273 | $4-Cl-C_6H_4-O-$ | $-(CH_2)_4-$ | $CH_3$ | $CH_3$ | | |
| 274 | $2,4-Cl_2-C_6H_3-O-$ | $-(CH_2)_4-$ | $CH_3$ | $CH_3$ | | |
| 275 | $2,6-Cl_2-C_6H_3-O-$ | $-(CH_2)_4-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 276 | $2-CH_3-C_6H_4-O-$ | $-(CH_2)_4-$ | $CH_3$ | $CH_3$ | | |
| 277 | $4-CH_3-C_6H_4-O-$ | $-(CH_2)_4-$ | $CH_3$ | $CH_3$ | | |
| 278 | $C_6H_5-O-$ | $-CH_2-CH_2-CH(CH_3)-CH_2-$ | $CH_3$ | $CH_3$ | | |
| 279 | $C_6H_5-O-$ | $-(CH_2)_5-$ | $CH_3$ | $CH_3$ | | |
| 280 | $3-Cl-C_6H_4-O-$ | $-(CH_2)_5-$ | $CH_3$ | $CH_3$ | | |
| 281 | $C_6H_5-O-$ | $-(CH_2)_3-CH(CH_3)-CH_2-$ | $CH_3$ | $CH_3$ | | |
| 282 | $C_6H_5-O-$ | $-(CH_2)_6-$ | $CH_3$ | $CH_3$ | | |
| 283 | $3-Cl-C_6H_4-O-$ | $-(CH_2)_6-$ | $CH_3$ | $CH_3$ | | |
| 284 | $C_6H_5-O-$ | $-(CH_2)_4-CH(CH_3)-CH_2-$ | $CH_3$ | $CH_3$ | | |
| 285 | A 1*) | - | $CH_3$ | $CH_3$ | | |
| 286 | A 2*) | - | $CH_3$ | $CH_3$ | | |
| 287 | A 3*) | - | $CH_3$ | $CH_3$ | | |
| 288 | A 4*) | - | $CH_3$ | $CH_3$ | | |
| 289 | A 5*) | - | $CH_3$ | $CH_3$ | | |
| 290 | A 6*) | - | $CH_3$ | $CH_3$ | | |
| 291 | A 7*) | - | $CH_3$ | $CH_3$ | | |
| 292 | A 8*) | - | $CH_3$ | $CH_3$ | | |
| 293 | A 9*) | - | $CH_3$ | $CH_3$ | | |
| 294 | A 10*) | - | $CH_3$ | $CH_3$ | | |
| 295 | A 11*) | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 296 | A 12*) | - | $CH_3$ | $CH_3$ | | |
| 297 | A 13*) | - | $CH_3$ | $CH_3$ | 116-119 | 2950, 1728, 1295, 1169 1069, 1020 |
| 298 | A 14*) | - | $CH_3$ | $CH_3$ | 103-106 | 2950, 1720, 1284, 1222, 1168, 1091, 1074, 1015, 756 |
| 299 | A 15*) | - | $CH_3$ | $CH_3$ | | |
| 300 | A 16*) | - | $CH_3$ | $CH_3$ | | |
| 301 | A 17*) | - | $CH_3$ | $CH_3$ | | |
| 302 | N-Pyrrolyl | $-CH(iso-C_3H_7)-$ | $CH_3$ | $CH_3$ | öl | 2970, 1743, 1275, 1222, 1069, 1019, 729 |
| 303 | 4-tert.-Butyl-$C_6H_4$ | $-CH_2-C(CH_3)=CH-CH=CH-$ | $CH_3$ | $CH_3$ | | |
| 304 | H | $-CH_2-CH(CH_3)-CH_2-CH(CH_3)-$ | $CH_3$ | $CH_3$ | | |
| 305 | H | $-CH_2-CH(CH_3)-CH_2-CH(C_2H_5)-$ | $CH_3$ | $CH_3$ | | |
| 306 | H | $-CH_2-CH(CH_3)-CH_2-CH(n-C_3H_7)-$ | $CH_3$ | $CH_3$ | | |
| 307 | H | $-CH_2-CH(CH_3)-CH_2-CH(i-C_3H_7)-$ | $CH_3$ | $CH_3$ | | |
| 308 | H | $-CH_2-C(CH_3)_2-CH_2-CH(CH_3)-CH_2-$ | $CH_3$ | $CH_3$ | | |
| 309 | H | $-(CH_2)_5-CH(C_2H_5)-$ | $CH_3$ | $CH_3$ | | |
| 310 | H | $-(CH_2)_5-CH(n-C_3H_7)-$ | $CH_3$ | $CH_3$ | | |
| 311 | H | $-(CH_2)_4-O-CH_2-C(CH_3)_2-$ | $CH_3$ | $CH_3$ | öl | 2957, 1731, 1220, 1145, 1069, 1020 |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 312 | H | $-CH_2-O-CH_2-C(CH_3)_2-$ | $CH_3$ | $CH_3$ | Öl | 2950, 1730, 1222, 1145, 1109, 1069, 1019 |
| 313 | $C_6H_5$ | $-CH=CH-(CH_2)_4-$ | $CH_3$ | $CH_3$ | | |
| 314 | H | $-CH_2-C(CH_3)_2-CH_2-CH(CH_3)_2-CH_2-$ | $CH_3$ | $CH_3$ | | |
| 315 | H | $-CH_2-CH(CH_3)-(CH_2)_2-CH(i-C_3H_7)-$ | $CH_3$ | $CH_3$ | | |
| 316 | 1-Methylcyclopropyl | - | $CH_3$ | $CH_3$ | 94 - 96 | 2980, 1739, 1714, 1302, 1171, 1069, 1011, 771. |
| 317 | 2-Methylcyclopropyl | - | $CH_3$ | $CH_3$ | | |
| 318 | 2-Phenylcyclopropyl | - | $CH_3$ | $CH_3$ | | |
| 319 | 1-Methylcyclohexyl | - | $CH_3$ | $CH_3$ | | |
| 320 | $4-Cl-C_6H_4$ | -CHCl- | $CH_3$ | $CH_3$ | | |
| 321 | 1-Methylcyclopropyl | - | $CH_3$ | $C_2H_5$ | | |
| 322 | 1-Methylcyclopropyl | - | $CH_3$ | $i-C_3H_7$ | | |
| 323 | 1-Methylcyclopropyl | - | $CH_3$ | $n-C_3H_7$ | | |
| 324 | 1-Methylcyclopropyl | - | $CH_3$ | $n-C_4H_9$ | | |
| 325 | 1-Methylcyclopropyl | - | $CH_3$ | $n-C_5H_{11}$ | | |
| 326 | 1-Methylcyclopropyl | - | H | $CH_3$ | | |
| 327 | 1-Methylcyclopropyl | - | $C_2H_5$ | $CH_3$ | | |
| 328 | 1-Methylcyclopropyl | - | $n-C_3H_7$ | $CH_3$ | | |
| 329 | 1-Methylcyclopropyl | - | $n-C_4H_9$ | $CH_3$ | | |
| 330 | 1-Methylcyclopropyl | - | $n-C_5H_{11}$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 331 | 2-Pyridyl | - | $CH_3$ | $CH_3$ | 123-126 | |
| 332 | 4-Methyl-2-pyridyl | - | $CH_3$ | $CH_3$ | | |
| 333 | 4-Fluoro-2-pyridyl | - | $CH_3$ | $CH_3$ | | |
| 334 | 4-Chloro-2-pyridyl | - | $CH_3$ | $CH_3$ | | |
| 335 | 6-Methyl-2-pyridyl | - | $CH_3$ | $CH_3$ | | |
| 336 | 6-Ethyl-2-pyridyl | - | $CH_3$ | $CH_3$ | | |
| 337 | 6-n-Propyl-2-pyridyl | - | $CH_3$ | $CH_3$ | | |
| 338 | 6-iso-Propyl-2-pyridyl | - | $CH_3$ | $CH_3$ | | |
| 339 | 6-n-Butyl-2-pyridyl | - | $CH_3$ | $CH_3$ | | |
| 340 | 6-Chloro-2-pyridyl | - | $CH_3$ | $CH_3$ | | |
| 341 | 3,6-Dichloro-2-pyridyl | - | $CH_3$ | $CH_3$ | | |
| 342 | 5-n-Butyl-2-pyridyl | - | $CH_3$ | $CH_3$ | | |
| 343 | 3-Pyridyl | - | $CH_3$ | $CH_3$ | | |
| 344 | 2-Methyl-3-pyridyl | - | $CH_3$ | $CH_3$ | | |
| 345 | 2-Chloro-3-pyridyl | - | $CH_3$ | $CH_3$ | | |
| 346 | 2-Fluoro-3-pyridyl | - | $CH_3$ | $CH_3$ | | |
| 347 | 4-Chloro-3-pyridyl | - | $CH_3$ | $CH_3$ | | |
| 348 | 5-Fluoro-3-pyridyl | - | $CH_3$ | $CH_3$ | | |
| 349 | 6-Chloro-3-pyridyl | - | $CH_3$ | $CH_3$ | | |
| 350 | 4-Pyridyl | - | $CH_3$ | $CH_3$ | | |
| 351 | 2-Methyl-4-pyridyl | - | $CH_3$ | $CH_3$ | | |
| 352 | 2,6-Dimethyl-4-pyridyl | - | $CH_3$ | $CH_3$ | | |
| 353 | 2,3-Dimethyl-4-pyridyl | - | $CH_3$ | $CH_3$ | | |
| 354 | 2-Phenyl-3-methyl-4-pyridyl | - | $CH_3$ | $CH_3$ | | |
| 355 | 3-Methyl-4-pyridyl | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 356 | 2-Ethyl-4-pyridyl | - | $CH_3$ | $CH_3$ | | |
| 357 | 2-n-Propyl-4-pyridyl | - | $CH_3$ | $CH_3$ | | |
| 358 | 2-n-Butyl-4-pyridyl | - | $CH_3$ | $CH_3$ | | |
| 359 | 2-Chloro-4-pyridyl | - | $CH_3$ | $CH_3$ | | |
| 360 | 2,6-Dichloro-4-pyridyl | - | $CH_3$ | $CH_3$ | | |
| 361 | 2-Chinolyl | - | $CH_3$ | $CH_3$ | | |
| 362 | 3-Chinolyl | - | $CH_3$ | $CH_3$ | | |
| 363 | 4-Chinolyl | - | $CH_3$ | $CH_3$ | | |
| 364 | 2-Methyl-4-chinolyl | - | $CH_3$ | $CH_3$ | | |
| 365 | 2-Ethyl-4-chinolyl | - | $CH_3$ | $CH_3$ | | |
| 366 | 2-n-Propyl-chinolyl | - | $CH_3$ | $CH_3$ | | |
| 367 | 2-iso-Propyl-4-chinolyl | - | $CH_3$ | $CH_3$ | | |
| 368 | 2-tert-Butyl-4-chinolyl | - | $CH_3$ | $CH_3$ | | |
| 369 | 2-Cyclohexyl-4-chinolyl | - | $CH_3$ | $CH_3$ | | |
| 370 | 3-Methyl-4-chinolyl | - | $CH_3$ | $CH_3$ | | |
| 371 | 3-Ethyl-4-chinolyl | - | $CH_3$ | $CH_3$ | | |
| 372 | 3-n-Propyl-4-chinolyl | - | $CH_3$ | $CH_3$ | | |
| 373 | 3-n-Butyl-4-chinolyl | - | $CH_3$ | $CH_3$ | | |
| 374 | 3-Phenyl-4-chinolyl | - | $CH_3$ | $CH_3$ | | |
| 375 | 3-Benzyl-4-chinolyl | - | $CH_3$ | $CH_3$ | | |
| 376 | 2-Methyl-3-acetyl-4-chinolyl | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 377 | 2-Phenyl-3-methoxy-4-chinolyl | - | $CH_3$ | $CH_3$ | | |
| 378 | 2-Methyl-3-cyano-4-chinolyl | - | $CH_3$ | $CH_3$ | | |
| 379 | 6-Methyl-4-chinolyl | - | $CH_3$ | $CH_3$ | | |
| 380 | 6-Chloro-4-chinolyl | - | $CH_3$ | $CH_3$ | | |
| 381 | 7-Chloro-4-chinolyl | - | $CH_3$ | $CH_3$ | | |
| 382 | 7-Chloro-8-methyl-3-chinolyl | - | $CH_3$ | $CH_3$ | | |
| 383 | 2,2-Dichlorcyclopropyl | - | $CH_3$ | $CH_3$ | | |
| 384 | 2-Furyl | -CH=CH- | $CH_3$ | $CH_3$ | 58-60 | |
| 385 | 1-Phenylcyclopropyl | - | $CH_3$ | $CH_3$ | | |
| 386 | 1-(2'Methylphenyl)-cyclopropyl | - | $CH_3$ | $CH_3$ | | |
| 387 | 1-(3'-Methylphenyl)-cyclopropyl | - | $CH_3$ | $CH_3$ | | |
| 388 | 1-(4'-Methylphenyl)-cyclopropyl | - | $CH_3$ | $CH_3$ | | |
| 389 | 1-(3',4'-Dimethylphenyl)-cyclopropyl | - | $CH_3$ | $CH_3$ | | |
| 390 | 1-(4'-tert.-Butylphenyl)-cyclopropyl | - | $CH_3$ | $CH_3$ | | |
| 391 | 1-(3'-Trifluormethylphenyl-cyclopropyl | - | $CH_3$ | $CH_3$ | | |
| 392 | 1-(2'-Fluorphenyl)-cyclopropyl | - | $CH_3$ | $CH_3$ | | |
| 393 | 1-(3'-Fluorphenyl)-cyclopropyl | - | $CH_3$ | $CH_3$ | | |
| 394 | 1-(4'-Fluorphenyl)-cyclopropyl | - | $CH_3$ | $CH_3$ | | |
| 395 | 1-(2'-Chlorphenyl)-cyclopropyl | - | $CH_3$ | $CH_3$ | | |
| 396 | 1-(3'-Chlorphenyl)-cyclopropyl | - | $CH_3$ | $CH_3$ | | |
| 397 | 1-(2',6'Dichlorphenyl)-cyclopropyl | - | $CH_3$ | $CH_3$ | | |
| 398 | 1-(3'4'-Dichlorphenyl)-cyclopropyl | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 399 | 1-(4'-Bromphenyl)-cyclopropyl | - | $CH_3$ | $CH_3$ | | |
| 400 | 1-(2'-Methoxyphenyl)-cyclopropyl | - | $CH_3$ | $CH_3$ | | |
| 401 | 1-(3'-Methoxyphenyl)-cyclopropyl | - | $CH_3$ | $CH_3$ | | |
| 402 | 1-(4'-Methoxyphenyl)-cyclopropyl | - | $CH_3$ | $CH_3$ | | |
| 403 | 1-(2',4'-Dimethoxyphenyl)-cyclopropyl | - | $CH_3$ | $CH_3$ | | |
| 404 | 1-(2',6'-Dimethoxyphenyl)-cyclopropyl | - | $CH_3$ | $CH_3$ | | |
| 405 | 1-(3',4'-Dimethoxyphenyl)-cyclopropyl | - | $CH_3$ | $CH_3$ | | |
| 406 | 1-(3',4'-Diethoxyphenyl)-cyclopropyl | - | $CH_3$ | $CH_3$ | | |
| 407 | 1-Trifluormethylcyclopropyl | - | $CH_3$ | $CH_3$ | 68 | 2970, 1746, 1724, 1404, 1145, 1110, 1012 |
| 408 | 1-Trimethylsilylcyclopropyl | - | $CH_3$ | $CH_3$ | 68 | 2950, 1719, 1713, 1273, 1153, 1067, 1017, 837 |
| 409 | 2-Furyl | - | $CH_3$ | $CH_3$ | 82-84 | |
| 410 | 2-Thiophenyl | - | $CH_3$ | $CH_3$ | 88-90 | |
| 411 | 2-Thiophenyl | -CH=CH- | $CH_3$ | $CH_3$ | 60 | |
| 412 | 2,6-$(OCH_3)_2)_2$-$C_6H_3$ | - | $CH_3$ | $CH_3$ | öl | 2950, 1733, 1597, 1476, 1257, 1113, 1069, 1018 |
| 413 | 2-F, 6-Cl-$C_6H_3$ | - | $CH_3$ | $CH_3$ | öl | 2950, 1738, 1450, 1270, 1069, 1019, 902, 791 |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 414 | 2-(N-Methyl)-pyrrolyl | - | $CH_3$ | $CH_3$ | Öl | 2960, 1726, 1705, 1412, 1322, 1244, 1104, 1069, 1018 |
| 415 | N-Pyrazolyl | $-CH(iso-C_3H_7)-$ | $CH_3$ | $CH_3$ | Öl | 2970, 1743, 1394, 1279, 1222, 1069, 1019, 754 |
| 416 | 1,2,4-Triazol-1-yl | $-CH(iso-C_3H_7)-$ | $CH_3$ | $CH_3$ | Öl | 2970, 1743, 1438, 1276, 1222, 1069, 1017, 957 |
| 417 | 1-Naphthyl | - | $CH_3$ | $CH_3$ | Öl | 2950, 1723, 1242, 1196, 1132, 1069, 1017, 784 |
| 418 | 9-Anthracenyl | - | $CH_3$ | $CH_3$ | Öl | 2950, 1725, 1199, 1069, 1016, 734 |
| 419 | 1-(4'-Chlorphenyl)-cyclobutyl | - | $CH_3$ | $CH_3$ | - | |
| 420 | $3-CF_3-C_6H_4$ | $-CH_2-$ | $CH_3$ | $CH_3$ | Öl | 2950, 1738, 1439, 1331, 1168, 1125, 1072, 1022 |
| 421 | $3,4-(OC_2H_5)_2-C_6H_3$ | $-CH_2-$ | $CH_3$ | $CH_3$ | Öl | 2980, 1738, 1514, 1261, 1223, 1142, 1069, 1019 |
| 422 | $3,4,5-(OCH_3)_3-C_6H_2$ | $-CH_2-$ | $CH_3$ | $CH_3$ | Öl | 2940, 1733, 1591, 1461, 1319, 1223, 1127, 1069, 1017 |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 423 | 4-F-$C_6H_4$ | $-C(CH_3)_2-CH_2-$ | $CH_3$ | $CH_3$ | öl | 2980, 1731, 1512, 1322, 1223, 1069, 1019, 835 |
| 424 | 4-F-$C_6H_4$ | $-C(CH_3)_2-CH(CH_3)-$ | $CH_3$ | $CH_3$ | öl | 2980, 1731, 1511, 1223, 1069, 1019, 836 |
| 425 | 2-$CH_3$, 6-$NO_2$-$C_6H_3$ | - | $CH_3$ | $CH_3$ | 87-89 | 2950, 1741, 1531, 1344, 1270, 1067, 758 |

*) Formeln siehe vorhergehenden Text

Die neuen Verbindungen zeichnen sich, allgemein ausgedrücket, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und könen als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwole, Soja, Kaffee, Zuckerrohr, Obst und Zierpflanzen im Gartenbau, Weinbau sowie Gemüse — wie Gurken, Bohnen und Kürbisgewächse —.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticilium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allemeinen zwischen 0,1 und 95, vorzugsweise zwischen 0,5 und 90 Gew.% Wirkstoff.

Die Aufwandmengen liegen je nach Art des gewünschten Effektes zwischen 0,02 un 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 316 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 316 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 316 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 316 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 316 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 316 werden mit 97 Gew.-Teilen feintiligem Kaolin innig vermischt. Man erhält auf diese Weise ein Stäubemittel, das 3 Gew.% des Wirkstoffs enthält.

VII. 30 Gew.-Teile der Verbindung Nr. 316 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 316 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 316 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,

Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thicarbamoyl)-disulfid;

Nitroderivate, wie
Dinitro-(1-methylheptyl)phenylcrotonat,
2-sec-Butyl-4,6-dinitrophenyl-3,3-dimethylacrylat,
2-sec-Butyl-4,6-dinitrophenyl-isoproylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-2-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid
5-Ethoxy-3-trichlormethyl-1,2,3-thiadiazol,

2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethylfuran-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-[3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

## Anwendungsbeispiele

Als Vergleichswirkstoff wurde
2-Benzyloxyphenyl-glyoxylsäuremethylester-O-methyloxim (A) — bekannt aus EP—253 213 — benutzt.

## Anwendungsbeispiel 1

Wirksamkeit gegen Pyrenophora teres

Gerstenkeimlinge der Sorte "Igri" wurden im Zweiblattstadium mit wäßrigen Suspensionen, die 80% Wirkstoff und 20% Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunde in eine Klimakammer mit hoher Luftfeuchtigkeit bei 18°C gestellt. Anschließend wurden die Pflanzen im Gewächshaus bei 20 bis 22°C und 70% relativer Luftfeuchtigkeit für weitere 5 Tage kultiviert. Dann wurde das Ausmaß der Symptomentwicklung ermittelt.

Das ergebnis zeigt, daß der Wirkstoff 316 bei der Anwendung als 0,05 %ige (Gew.%) Spritzbrühe eine bessere fungizide Wirkung zeigt (95%) als der bekannte Vergleichswirkstoff A (80%).

**Patentansprüche**

1. Oximether der allgemeinen Formel I

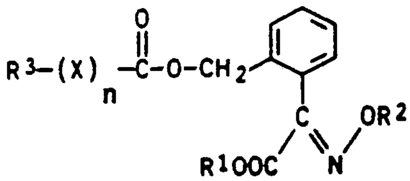

(I)

in der

$R^1$ und $R^2$ Wasserstoff oder $C_1$—$C_5$-Alkyl,

$R^3$ Wasserstoff, Halogen, Cyano, Aryl, Aryloxy, wobei der aromatische Ring gegebenenfalls substituiert ist durch $C_1$—$C_6$-Alkyl, $C_2$—$C_4$-Alkenyl, $C_1$—$C_2$-Halogenalkyl, $C_1$—$C_6$-Alkoxy, $C_1$—$C_4$-Alkoxy-$C_1$—$C_4$-alkyl, Aryl, Aryl-$C_1$—$C_2$-alkyl, Aryloxy, Aryloxy-$C_1$—$C_4$-alkyl, Aryloxy-$C_1$—$C_4$-alkoxy, Halogenaryloxy-$C_1$—$C_4$-alkoxy, Halogen, Halogen-$C_1$—$C_4$-alkoxy, $C_1$—$C_4$-Alkylthio, Thiocyanato, Cyano, Nitro, oder $R^3$ Heteroaryl, Adamantyl, Fluorenyl oder einen $C_3$—$C_7$-Cycloalkylrest oder $C_5$—$C_6$-Cycloalkenylrest bedeutet, wobei diese Reste gegebenenfalls substituiert sind durch $C_1$—$C_4$-Alkyl Halogen, $C_1$—$C_2$-Halogenalkyl, $C_3$—$C_4$-Alkenyl, Acetyl, $C_2$—$C_4$-Halogenalkenyl, Methoxycarbonyl-$C_3$—$C_4$-Alkenyl, Phenyl, Cyclopentylidenmethyl, Halogenphenyl, $C_1$—$C_2$-Alkoxyphenyl, $C_1$—$C_4$-Alkylphenyl,

X einen, gegebenenfalls durch Halogen oder Hydroxy substituierten, gegebenenfalls ungesättigten $C_1$—$C_{12}$-Alkylenrest bedeutet und

n die Zahlen 0 oder 1 bedeutet,

2. Verfahren zur Bekämpfung von Pilzen, dadurch gekennzeichnet, daß man die Pilze oder die von Pilzebefall bedrohten Materialien, Pflanzen, Saatgüter oder den Erdboden mit einer fungizid wirksamen Menge einer Verbindung der Formel I

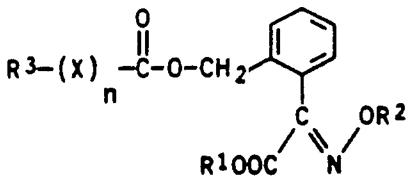

(I)

in der

$R^1$ und $R^2$ Wasserstoff oder $C_1$—$C_5$-Alkyl,

$R^3$ Wasserstoff, Halogen, Cyano, Aryl, Aryloxy, wobei der aromatische Ring gegebenenfalls substituiert ist durch $C_1$—$C_6$-Alkyl, $C_2$—$C_4$-Alkenyl, $C_1$—$C_2$-Halogenalkyl, $C_1$—$C_6$-Alkoxy, $C_1$—$C_4$-Alkoxy-$C_1$—$C_4$-alkyl, Aryl, Aryl-$C_1$—$C_2$-alkyl, Aryloxy, Ayloxy-$C_1$—$C_4$-alkyl, Aryloxy-$C_1$—$C_4$-alkoxy, Halogenaryloxy-$C_1$—$C_4$-alkoxy, Halogen, Halogen-$C_1$—$C_4$-alkoxy, $C_1$—$C_4$-Alkylthio, Thiocyanato, Cyano, Nitro, oder $R^3$ Heteroaryl, Adamantyl, Fluorenyl oder einen $C_3$—$C_7$-Cycloalkylrest oder $C_5$—$C_6$-Cycloalkenylrest bedeutet, wobei diese Reste gegebenenfalls substituiert sind durch $C_1$—$C_4$-Alkyl, Methyl, Ethyl, Halogen, $C_1$—$C_2$-Halogenalkyl, $C_3$—$C_4$-Alkenyl $C_2$—$C_4$-Halogenalkenyl, Methoxycarbonyl-$C_3$—$C_4$-Alkenyl, Acetyl, Cyclopentylidenmethyl, Halogenphenyl, Phenyl, $C_1$—$C_2$-Alkoxyphenyl, $C_1$—$C_4$-Alkylphenyl, Phenyl,

X einen, gegebenenfalls durch Halogen oder Hydroxy substituierten, gegebenenfalls ungesättigten $C_1$—$CX_{12}$-Alkylenrest bedeutet und

n die Zahlen 0 oder 1 bedeutet, behandelt.

3. Fungizid, enthaltend einen inerten Trägerstoff und eine fungizid wirksame Menge einer Verbindung der Formel I

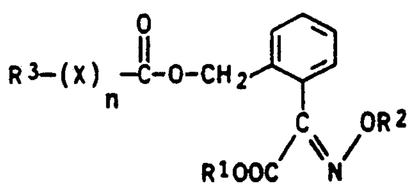

(I)

in der

$R^1$ und $R^2$ Wasserstoff oder $C_1$—$C_5$-Alkyl,

$R^3$ Wasserstoff, Halogen, Cyano, Aryl, Aryloxy, wobei der aromatische Ring gegebenenfalls substituiert ist durch $C_1$—$C_6$-Alkyl, $C_2$—$C_4$-Alkenyl, $C_1$—$C_2$-Halogenalkyl, $C_1$—$C_6$-Alkoxy, $C_1$—$C_4$-Alkoxy-$C_1$—$C_4$-alkyl, Aryl, Aryl-$C_1$—$C_2$-alkyl, Aryloxy, Aryloxy-$C_1$—$C_4$-alkyl, Aryloxy-$C_1$—$C_4$-alkoxy, Halogenaryloxy-$C_1$—$C_4$-alkoxy, Halogen, Halogen-$C_1$—$C_4$-alkoxy, $C_1$—$C_4$-Alkylthio, Thiocyanato, Cyano, Nitro, oder $R^3$ Heteroaryl, Adamantyl, Fluorenyl oder einen $C_3$—$C_7$-Cycloalkylrest oder $C_5$—$C_6$-Cycloalkenylrest bedeutet, wobei diese Reste gegebenenfalls substituiert sind durch $C_1$—$C_4$-Alkyl, Methyl, Ethyl, Halogen, $C_1$—$C_2$-Halogenalkyl, $C_3$—$C_4$-Alkenyl, Acetyl, $C_2$—$C_4$-Halogenalkenyl, Methoxycarbonyl-$C_3$—$C_4$-Alkenyl, Phenyl, Cyclopentylidenmethyl, Halogenphenyl, $C_1$—$C_2$-Alkoxyphenyl, $C_1$—$C_4$-Alkylphenyl,

X einen, gegebenenfalls durch Halogen oder Hydroxy substituierten, gegebenenfalls ungesättigten $C_1$—$C_{12}$-Alkylenrest bedeutet und

n die Zahlen 0 oder 1 bedeutet,

4. Verfahren zur Herstellung eines fungiziden Mittels, dadurch gekennzeichnet, daß man eine oder mehrere Verbindungen der Formel I gemäß Anspruch 1 mit einem festen flüssigen Trägerstoff sowie gegebenenfalls mit einem oder mehreren oberflächenaktiven Mitteln mischt.

5. Verbindung gemäß Formel I in Anspruch 1, dadurch gekennzeichnet, daß $R^1$ und $R^2$ Methyl, $R^3$ 1-Methylcyclopropyl und n die Zahl 0 bedeutet.

6. Verbindung der Formel I gemäß Anspruch 1, dadurch gekennzeichnet, daß $R^1$ und $R^2$ Methyl, $R^3$ 1-(4-Chlorphenyl)-cyclopropyl und n die Zahl 0 bedeutet.

7. Verbindung der Formel I gemäß Anspruch 1, dadurch gekennzeichnet, daß $R^1$ und $R^2$ Methyl, $R^3$ 1-(2,4-Chlorphenyl)-cyclopropyl und n die Zahl 0 bedeutet.

8. Verfahren zur Herstellung eines Oximethers der allgemeinen Formel I

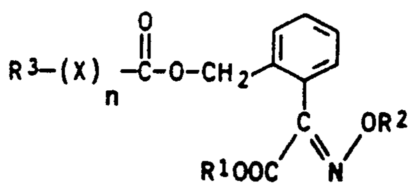

(I)

in der

$R^1$ und $R^2$ Wasserstoff oder $C_1$—$C_5$-Alkyl,

$R^3$ Wasserstoff, Halogen, Cyano, Aryl, Aryloxy, wobei der aromatische Ring gegebenenfalls substituiert ist durch $C_1$—$C_6$-Alkyl, $C_2$—$C_4$-Alkenyl, $C_1$—$C_2$-Halogenalkyl, $C_1$—$C_6$-Alkoxy, $C_1$—$C_4$-Alkoxy-$C_1$—$C_4$-alkyl, Aryl, Aryl-$C_1$—$C_2$-alkyl, Aryloxy, Aryloxy-$C_1$—$C_4$-alkyl, Aryloxy-$C_1$—$C_4$-alkoxy, Halogenaryloxy-$C_1$—$C_4$-alkoxy, Halogen, Halogen-$C_1$—$C_4$-alkoxy, $C_1$—$C_4$-Alkylthio, Thiocyanato, Cyano, Nitro, oder $R^3$ Heteroaryl, Adamantyl, Fluorenyl oder einen $C_3$—$C_7$-Cycloalkylrest oder $C_5$—$C_6$-Cycloalkenylrest bedeutet, wobei diese Reste gegebenenfalls substituiert sind durch $C_1$—$C_4$-Alkyl, Halogen, $C_1$—$C_2$-Halogenalkyl, $C_3$—$C_4$-Alkenyl, Acetyl, $C_2$—$C_4$-Halogenalkenyl, Methoxycarbonyl-$C_3$—$C_4$-Alkenyl, Phenyl, Cyclopentylidenmethyl, Halogenphenyl, $C_1$—$C_2$-Alkoxyphenyl, $C_1$—$C_4$-Alkylphenyl,

X einen, gegebenenfalls durch Halogen oder Hydroxy substituierten, gegebenenfalls ungesättigten $C_1$—$C_{12}$-Alkylenrest bedeutet und

n die Zahlen 0 oder 1 bedeutet, dadurch gekennzeichnet, daß man eine Brommethylverbindung de Formel III

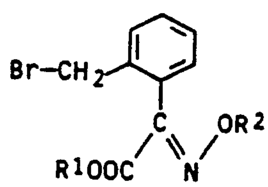

(III)

in der $R^1$ und $R^2$ die oben genannten Bedeutungen haben, umsetzt mit einem Carbonsäuresalz der Formel II

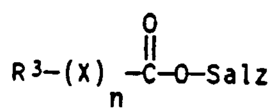

(II)

in der $R^3$, X und n die oben genannten Bedeutungen haben, und Salz Na, K oder Ammonium bedeutet.

**Revendications**

1. Ether d'oxime de la formule générale I

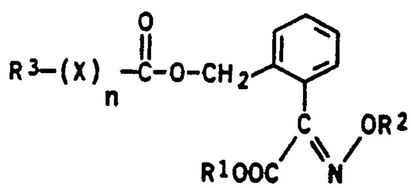

(I)

dans laquelle

$R^1$ et $R^2$ représentent hydrogène ou alkyle en $C_1$—$C_5$

$R^3$, hydrogène, halogène, cyano, aryle, aryloxy, la chaîne aromatique étant éventuellement substituée par alkyle en $C_1$—$C_6$, alcényle en $C_2$—$C_4$, halogènalkyle en $C_1$—$C_2$, alcoxy en $C_1$—$C_6$, alcoxy en $C_1$—$C_4$-alkyle

en $C_1$—$C_4$, aryle, aryl-alkyle en $C_1$—$C_2$, aryloxy, aryloxy-alkyle en $C_1$—$C_4$, aryloxy-alcoxy en $C_1$—$C_4$, halogènaryloxy-alcoxy en $C_1$—$C_4$, halogène, halogèn-alcoxy en $C_1$—$C_4$, alkylthio en $C_1$—$C_4$, thiocyanato, cyano, nitro, ou $R^3$ représente hétéroaryle, adamantyle, fluorényle ou un reste cycloalkyle en $C_3$—$C_7$ ou un reste cycloalcényle en $C_5$—$C_6$, ces restes étant éventuellement substitués par alkyle en $C_1$—$C_4$, halogène, halogénalkyle en $C_1$—$C_2$, alcényle en $C_3$—$C_4$, acétyle, halogènalcényle en $C_2$—$C_4$, méthoxycarbonyl-alcényle en $C_3$—$C_4$, phényle, cyclopentylidenméthyle, halogénophényle, alcoxyphényle en $C_1$—$C_{12}$, alkylphényle en $C_1$—$C_4$

X représente un reste alkylène en $C_1$—$C_{12}$ éventuellement insaturé, substitué éventuellement par halogène ou hydroxy, et

n représente les nombres 0 et 1.

2. Procédé de lutte contre les champignons, caractérisé par le fait qu'on traite les champignons ou les matériaux, plantes, semences ou le sol avec une quantité efficace du pont de vue fongicide d'un composé de la formule I

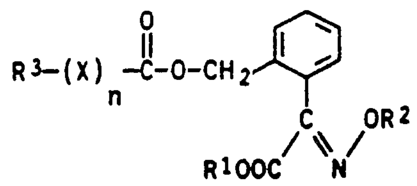

(I)

dans laquelle

$R^1$ et $R^2$ représentent hydrogène ou alkyle en $C_1$—$C_5$

$R^3$, hydrogène, halogène, cyano, aryle, aryloxy, la chaîne aromatique étant éventuellement substituée par alkyle en $C_1$—$C_6$, alcényle en $C_2$—$C_4$, halogènalkyle en $C_1$—$C_2$, alcoxy en $C_1$—$C_6$, alcoxy en $C_1$—$C_4$-alkyle en $C_1$—$C_4$, aryle, aryl-alkyle en $C_1$—$C_2$, aryloxy, aryloxy-alkyle en $C_1$—$C_4$, aryloxy-alcoxy en $C_1$—$C_4$, halogènaryloxy-alcoxy en $C_1$—$C_4$, halogène, halogèn-alcoxy en $C_1$—$C_4$, alkylthio en $C_1$—$C_4$, thiocyanato, cyano, nitro, ou $R^3$ représente hétéroaryle, adamantyle, fluorényle ou un reste cycloalkyle en $C_3$—$C_7$ ou un reste cycloalcényle en $C_5$—$C_6$, ces restes étant éventuellement substitués par alkyle en $C_1$—$C_4$, halogène, halogénalkyle en $C_1$—$C_2$, alcényle en $C_3$—$C_4$, acétyle, halogènalcényle en $C_2$—$C_4$, méthoxycarbonyl-alcényle en $C_3$—$C_4$, phényle, cyclopentylidenméthyle, halogénophényle, alcoxyphényle en $C_1$—$C_2$, alkylphényle en $C_1$—$C_4$

X représente un reste alkylène en $C_1$—$C_{12}$ éventuellement insaturé, substitué éventuellement par halogène ou hydroxy, et

n représente les nombres 0 et 1.

3. Fongicide contenant un support inerte et une quantité efficace du point de vue fongicide d'un composé de la formule I

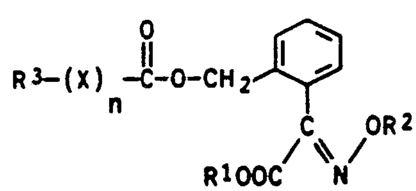

(I)

dans laquelle

$R^1$ et $R^2$ représentent hydrogène ou alkyle en $C_1$—$C_5$

$R^3$, hydrogène, halogène, cyano, aryle, aryloxy, la chaîne aromatique étant éventuellement substituée par alkyle en $C_1$—$C_6$, alcényle en $C_2$—$C_4$, halogènalkyle en $C_1$—$C_2$, alcoxy en $C_1$—$C_6$, alcoxy en $C_1$—$C_4$-alkyle en $C_1$—$C_4$, aryle, aryl-alkyle en $C_1$—$C_2$, aryloxy, aryloxy-alkyle en $C_1$—$C_4$, aryloxy-alcoxy en $C_1$—$C_4$, halogènaryloxy-alcoxy en $C_1$—$C_4$, halogène, halogèn-alcoxy en $C_1$—$C_4$, alkylthio en $C_1$—$C_4$, thiocyanato, cyano, nitro, ou $R^3$ représente hétéroaryle, adamantyle, fluorényle ou un reste cycloalkyle en $C_3$—$C_7$ ou un reste cycloalcényle en $C_5$—$C_6$, ces restes étant éventuellement substitués par alkyle en $C_1$—$C_4$, halogène, halogénalkyle en $C_1$—$C_2$, alcényle en $C_3$—$C_4$, acétyle, halogènalcényle en $C_2$—$C_4$, méthoxycarbonyl-alcényle en $C_3$—$C_4$, phényle, cyclopentylidenméthyle, halogénophényle, alcoxyphényle en $C_1$—$C_2$, alkylphényle en $C_1$—$C_4$

X représente un reste alkylène en $C_1$—$C_{12}$ éventuellement insaturé, substitué éventuellement par halogène ou hydroxy, et

n représente les nombres 0 et 1.

4. Procédé de préparation d'un agent fongicide, caractérisé par le fait qu'on mélange un ou plusieurs composés de la formule I selon la revendication 1 avec un support solide ou liquide ainsi qu'avec éventuellement un ou plusieurs agents tensioactifs.

5. Composé de la formule I selon la revendication 1, caractérisé par le fait que $R^1$ et $R^2$ représentent méthyle, $R^3$, 1-methylcyclopropyle et n le chiffre 0.

6. Composé de la formule I selon la revendication 1, caractérisé par le fait que $R^1$ et $R^2$ représentent méthyle, $R^3$, 1-(4-chlorophényl)-cyclopropyle et n le chiffre 0.

7. Composé de la formule I selon la revendication 1, caractérisé par le fait que $R^1$ et $R^2$ représentent méthyle, $R^3$, 1-(2,4-dichlorophényl)-cyclopropyle et n le chiffre 0.

8. Procédé de préparation d'un éther d'oxime de la formule générale I

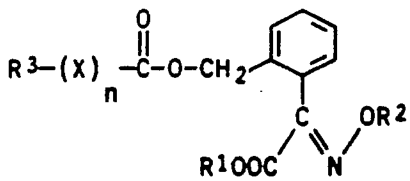

(I)

dans laquelle

$R^1$ et $R^2$ représentent hydrogène ou alkyle en $C_1$—$C_5$

$R^3$, hydrogène, halogène, cyano, aryle, aryloxy, la chaîne aromatique étant éventuellement substituée par alkyle en $C_1$—$C_6$, alcényle en $C_2$—$C_4$, halogènalkyle en $C_1$—$C_2$, alcoxy en $C_1$—$C_6$, alcoxy en $C_1$—$C_4$-alkyle en $C_1$—$C_4$, aryle, aryl-alkyle en $C_1$—$C_2$, aryloxy, aryloxy-alkyle en $C_1$—$C_4$, aryloxy-alcoxy en $C_1$—$C_4$, halogènaryloxy-alcoxy en $C_1$—$C_4$, halogène, halogèn-alcoxy en $C_1$—$C_4$, alkylthio en $C_1$—$C_4$, thiocyanato, cyano, nitro, ou $R^3$ représente hétéroaryle, adamantyle, fluorényle ou un reste cycloalkyle en $C_3$—$C_7$ ou un reste cycloalcényle en $C_5$—$C_6$, ces restes étant éventuellement substitués par alkyle en $C_1$—$C_4$, halogène, halogénalkyle en $C_1$—$C_2$, alcényle en $C_3$—$C_4$, acétyle, halogènalcényle en $C_2$—$C_4$, méthoxycarbonyl-alcényle en $C_3$—$C_4$, phényle, cyclopentylidenméthyle, halogénophényle, alcoxyphényle en $C_1$—$C_2$, alkylphényle en $C_1$—$C_4$

X représente un reste alkylène en $C_1$—$C_{12}$ éventuellement insaturé, substitué éventuellement par halogène ou hydroxy, et

n représente les nombres 0 et 1.

caractérisé par le fait qu'on fait réagir un composé bromométhyle de la formule III

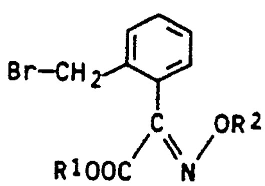

(III)

dans laquelle $R^1$ et $R^2$ ont les significations susindiquées, avec un sel d'acide carboxylique de la formule II

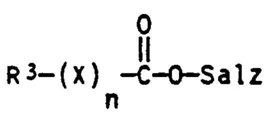

(II)

dans laquelle $R^3$, X et n ont les significations susindiquées et sel représente Na, K ou ammonium.

**Claims**

1. Oxime ethers of the general formula I

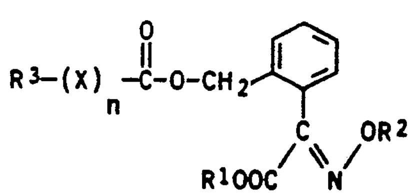

(I)

where

$R^1$ and $R^2$ are each hydrogen or $C_1$—$C_5$-alkyl,

$R^3$ is hydrogen, halogen, cyano, aryl or aryloxy, the aromatic ring being unsubstituted or substituted by $C_1$—$C_6$-alkyl, $C_2$—$C_4$-alkenyl, $C_1$—$C_2$-haloalkyl, $C_1$—$C_6$-alkoxy, $C_1$—$C_4$-alkoxy-$C_1$—$C_4$-alkyl, aryl, aryl-$C_1$—$C_2$-alkyl, aryloxy, aryloxy-$C_1$—$C_4$-alkyl, aryloxy-$C_1$—$C_4$-alkoxy, haloaryloxy-$C_1$—$C_4$-alkoxy, halogen, halogen-$C_1$—$C_4$-alkoxy, $C_1$—$C_4$-alkylthio, thiocyanato, cyano or nitro, or $R^3$ is heteroaryl, adamantyl, fluorenyl or $C_3$—$C_7$-cycloalkyl or $C_5$—$C_6$-cycloalkenyl, these radicals being unsubstituted or substituted by $C_1$—$C_4$-alkyl, halogen, $C_1$—$C_2$-haloalkyl, $C_3$—$C_4$-alkenyl, acetyl, $C_2$—$C_4$-haloalkenyl, methoxycarbonyl-$C_3$—$C_4$-alkenyl, phenyl, cyclopentylidenemethyl, halophenyl, $C_1$—$C_2$-alkoxyphenyl or $C_1$—$C_4$-alkylphenyl,

X is saturated or unsaturated $C_1$—$C_{12}$-alkylene which is unsubstituted or substituted by halogen or hydroxy, and

n is 0 or 1.

2. A process for combating fungi, wherein the fungi, or the materials, plants, seed or the soil to be protected against fungus attack are treated with a fungicidally effective amount of a compound of the formula I

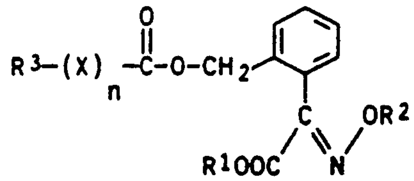

(I)

where

$R^1$ and $R^2$ are each hydrogen or $C_1$—$C_5$-alkyl,

$R^3$ is hydrogen, halogen, cyano, aryl or aryloxy, the aromatic ring being unsubstituted or substituted by $C_1$—$C_6$-alkyl, $C_2$—$C_4$-alkenyl, $C_1$—$C_2$-haloalkyl, $C_1$—$C_6$-alkoxy, $C_1$—$C_4$-alkoxy-$C_1$—$C_4$-alkyl, aryl, aryl-$C_1$—$C_2$-alkyl, aryloxy, aryloxy-$C_1$—$C_4$-alkyl, aryloxy-$C_1$—$C_4$-alkoxy, haloaryloxy-$C_1$—$C_4$-alkoxy, halogen, halogen-$C_1$—$C_4$-alkoxy, $C_1$—$C_4$-alkylthio, thiocyanato, cyano or nitro, or $R^3$ is heteroaryl, adamantyl, fluorenyl or $C_3$—$C_7$-cycloalkyl or $C_5$—$C_6$-cycloalkenyl, these radicals being unsubstituted or substituted by $C_1$—$C_4$-alkyl, methyl, ethyl, halogen, $C_1$—$C_2$-haloalkyl, $C_3$—$C_4$-alkenyl, $C_2$—$C_4$-haloalkenyl, methoxycarbonyl-$C_3$—$C_4$-alkenyl, phenyl, cyclopentylidenemethyl, halophenyl, $C_1$—$C_2$-alkoxyphenyl or $C_1$—$C_4$-alkylphenyl or phenyl,

X is saturated or unsaturated $C_1$—$C_{12}$-alkylene which is unsubstituted or substituted by halogen or hydroxy, and

n is 0 or 1.

3. A fungicide containing an inert carrier and a fungicidally effective amount of a compound of the formula I

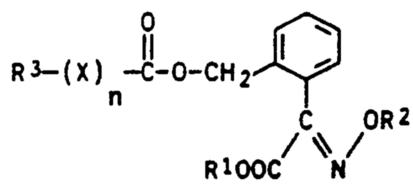

(I)

where

$R^1$ and $R^2$ are each hydrogen or $C_1$—$C_5$-alkyl,

$R^3$ is hydrogen, halogen, cyano, aryl or aryloxy, the aromatic ring being unsubstituted or substituted by $C_1$—$C_6$-alkyl, $C_2$—$C_4$-alkenyl, $C_1$—$C_2$-haloalkyl, $C_1$—$C_6$-alkoxy, $C_1$—$C_4$-alkoxy-$C_1$—$C_4$-alkyl, aryl, aryl-$C_1$—$C_2$-alkyl, aryloxy, aryloxy-$C_1$—$C_4$-alkyl, aryloxy-$C_1$—$C_4$-alkoxy, haloaryloxy-$C_1$—$C_4$-alkoxy, halogen, halogen-$C_1$—$C_4$-alkoxy, $C_1$—$C_4$-alkylthio, thiocyanato, cyano or nitro, or $R^3$ is heteroaryl, adamantyl, fluorenyl or $C_3$—$C_7$-cycloalkyl or $C_5$—$C_6$-cycloalkenyl, these radicals being unsubstituted or substituted by $C_1$—$C_4$-alkyl, methyl, ethyl, halogen, $C_1$—$C_2$-haloalkyl, $C_3$—$C_4$-alkenyl, acetyl, $C_2$—$C_4$-haloalkenyl, methoxycarbonyl-$C_3$—$C_4$-alkenyl, phenyl, cyclopentylidenemethyl, halophenyl, $C_1$—$C_2$-alkoxyphenyl or $C_1$—$C_4$-alkylphenyl,

X is saturated or unsaturated $C_1$—$C_{12}$-alkylene which is unsubstituted or substituted by halogen or hydroxy, and

n is 0 or 1.

4. A process for preparing a fungicide, wherein one or more compounds of the formula I as claimed in claim 1 is/are mixed with a solid or liquid carrier and, if required, one or more surface-active agents.

5. A compound of the formula I as claimed in claim 1, where $R^1$ and $R^2$ are methyl, $R^3$ is 1-methylcyclopropyl and n is 0.

6. A compound of the formula I as claimed in claim 1, where $R^1$ and $R^2$ are methyl, $R^3$ is 1-(4-chlorophenyl)-cyclopropyl and n is 0.

7. A compound of the formula I as claimed in claim 1, where $R^1$ and $R^2$ are methyl, $R^3$ is 1-(2,4-dichlorophenyl)-cyclopropyl and n is 0.

8. A process for preparing oxime ethers of the general formula I

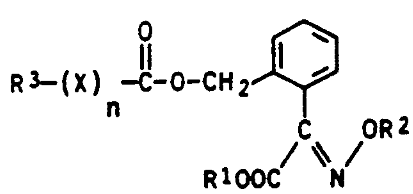

(I)

where

$R^1$ and $R^2$ are each hydrogen or $C_1$—$C_5$-alkyl,

$R^3$ is hydrogen, halogen, cyano, aryl or aryloxy, the aromatic ring being unsubstituted or substituted by $C_1$—$C_6$-alkyl, $C_2$—$C_4$-alkenyl, $C_1$—$C_2$-haloalkyl, $C_1$—$C_6$-alkoxy, $C_1$—$C_4$-alkoxy-$C_1$—$C_4$-alkyl, aryl, aryl-$C_1$—$C_4$-alkyl, aryloxy, aryloxy-$C_1$—$C_4$-alkyl, aryloxy-$C_1$—$C_4$-alkoxy, haloaryloxy-$C_1$—$C_4$-alkoxy, halogen, halogen-$C_1$—$C_4$-alkoxy, $C_1$—$C_4$-alkylthio, thiocyanato, cyano or nitro, or $R^3$ is heteroaryl, adamantyl, fluorenyl or

$C_3$—$C_7$-cycloalkyl or $C_5$—$C_6$-cycloalkenyl, these radicals being unsubstituted or substituted by $C_1$—$C_4$-alkyl, halogen, $C_1$—$C_2$-haloalkyl, $C_3$—$C_4$-alkenyl, acetyl, $C_2$—$C_4$-haloalkenyl, methoxycarbonyl-$C_3$—$C_4$-alkenyl, phenyl, cyclopentylidenemethyl, halophenyl, $C_1$—$C_2$-alkoxyphenyl or $C_1$—$C_4$-alkylphenyl,

X is saturated or unsaturated $C_1$—$C_{12}$-alkylene which is unsubstituted or substituted by halogen or hydroxy, and

n is 0 or 1, wherein a bromomethyl compound of the formula III

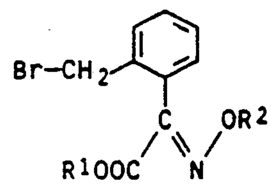

(III)

where $R^1$ and $R^2$ have the abovementioned meanings, is reacted with a carboxylate of the formula II

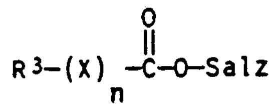

(II)

where $R^3$, X and n have the abovementioned meanings and Salt is Na, K or ammonium.